# EUROPEAN PATENT APPLICATION

(11) **EP 2 251 694 A1**
(43) Date of publication of application: **17.11.2010**
(21) Application number: 09722615.3
(22) Date of filing: 28.01.2009
(51) Int. Cl.: G01N 33/574, A61K 47/42, C12Q 1/04

(54) **KIT FOR DETECTING CANCER CELLS METASTASIZING INTO SENTINEL LYMPH NODE**

(30) Priority: 29.02.2008 JP 2008050735
(71) Applicant: Shinshu University, Matsumoto-shi, Nagano 390-8621 (JP)
(72) Inventor: OHHASHI, Toshio, Matsumoto-shi Nagano 390-8621 (JP); KAWAI, Yoshiko, Matsumoto-shi Nagano 390-8621 (JP)
(74) Representative: von Kreisler Selting Werner
(86) International application number: PCT/JP2009/051385
(87) International publication number: WO 2009/116322

(57) **Abstract**

Provided is a simple kit by which carcinoma cells metastasizing from a primary cancer lesion to a lymph node, in particular, a sentinel lymph node can be conveniently and accurately detected within a short period of time. Also disclosed is a drug delivery agent whereby a drug to be used in a test or therapy can be preferentially delivered into the sentinel lymph node as described above in which an environment easily allowing the adhesion of micro cancer cells has been constructed by a primary cancer lesion and to which micro cancer cells adhere. A kit for detecting cancer cells metastasizing into a sentinel lymph node which comprises a human lymph channel-origin endothelial cell line attached to a base material. A drug delivery agent toward to sentinel lymph node wherein an antibody against an adhesive molecule, which mediates the adhesion of metastatic cancer cells from a primary cancer lesion to lymph channel endothelial cells in the sentinel lymph node, is exposed on the surface of colloidal particles.

## Description

### Technical Field

The present invention relates to a kit for detecting carcinoma cells lymphogenously metastasizing from a primary tumor to a lymph node, in particular a sentinel lymph node, and also relates to a drug delivery agent whereby the drug can be preferentially delivered to the sentinel lymph node.

### Background of the Invention

Metastasis of carcinoma cells mainly occurs through a lymphatic system. As a treatment for a patient with cancer, surgery of removing a primary tumor and also a lymph node to which carcinoma cells may be metastasized is performed. The operative procedure of removing the lymph node is complicated; therefore, such surgery can be a heavy burden on the patient physically. In addition, after the removal, it is necessary to perform a clinical assay of criterion for metastasis of lymph nodes to examine whether the lymphatic system with the metastatic cancer cells is completely removed and also to examine the possibility of another cancer metastasis to the lymphatic system.

To remove only a primary tumor and a lymph node to be removed by surgery, a clinical assay method in which a sentinel lymph node (SLN) of a regional lymph node, which is the first lymph node in the lymphatic network draining from the primary tumor, is mapped by injecting radioisotopes or dye to the primary tumor to proceed biopsy, has been proposed.

The SLN is the presumptive initial site of lymphatic micro-metastasis of carcinoma cells. The clinical importance of examining the SLN has been proven in many breast cancer patients; however, the biological and histological properties of lymphatic endothelial cells (LECs) in the SLN and the nearest afferent lymph vessels thereof that can interact with micro-metastatic carcinoma cells remain unclear.

Recently, it has become known that primary tumors influence microenvironment of tumor tissues before metastasis. For example, matrix metalloproteinase 9, which is expressed in lung macrophages and endothelial cells in response to the primary tumors, and promotes the invasion of tumor cells into lung tissues and the induction thereof in a premetastatic phase, was dependent on VEGF-A secreted from the primary tumors. However, it is unclear which molecule in the prometastatic SLN induces a suitable environment for micrometastasis that relates to attachment of carcinoma cells and the LECs. Therefore, a method for detecting the micro-metastasis of the carcinoma cells or suppressing the metastasis of carcinoma cells by inhibiting the micro-metastasis of carcinoma cells is desired.

As disclosed in Japanese Unexamined Patent Publication No.2007-222155 and Kawai Y., et al., Lymphatic Research and Biology, 2007, Vol.5; pp. 115-126, the inventors of the present invention established a human lymphatic endothelial cell line from afferent lymph vessels of the SLN in breast cancer patients by using protease.

Using human breast carcinoma cells, MDA-MB-231 or MCF-7, the inventors of the present invention examined the effects of supernatants cultured with the cell lines on the expression of adhesion molecules on human LECs and then investigated whether the expressed adhesion molecules can accelerate the attachment of the carcinoma cells on the human LECs. The inventors also examined the possibility of which the carcinoma cells, in particular malignant breast carcinoma cells, can release chemical substances that make a prometastatic environment suitable for micrometastasis of the carcinoma cells in the SLN and the nearest afferent lymph vessels thereof. Also, the inventors examined the effects of various kinds of chemokines on the expression of adhesion molecules on the cultured human LECs located in the nearest afferent lymph vessels of the SLN and then investigated whether the expressed adhesion molecules are able to facilitate the attachment of the carcinoma cells to the LECs. In addition, the inventors also studied the immunohistochemical expression of the adhesion molecules on frozen tissues of the SLN isolated freshly from breast cancer patients. Based on the results of these studies, the inventors accomplished the present invention.

### Summary of the Invention

The present invention has been developed to solve the before-mentioned problems. And it is the object of the present invention to provide a kit for simply and accurately detecting carcinoma cells lymphogenously metastasizing from a primary tumor to a lymph node, in particular SLN, within a short period of time. The other object of the present invention is to provide a drug delivery agent whereby drug used for diagnosis and medical treatment can be preferentially delivered to the SLN to which micro carcinoma cells are attached due to a suitable environment for micrometastasis in response to the primary tumors.

The kit for detecting carcinoma cells metastasizing to SLN developed to solve the before-mentioned objects preferably comprises an endothelial cell line derived from a human lymph vessel which is applied onto a medium.

In the kit for detecting carcinoma cells metastasizing to SLN, the endothelial cell line derived from the human sentinel lymph vessel is consisted of endothelial cells collected through abrasion by intraluminal circulation of protease solution in an extirpated human lymph vessel.

The kit for detecting carcinoma cells metastasizing to SLN further comprises an immunoassay detecting agent, which detects an adhesive molecule mediated attachment of the carcinoma cells metastasized from a primary tumor with the endothelial cell line derived from the human lymph vessel by performing an antigen-antibody reaction.

In the kit for detecting carcinoma cells metastasizing to SLN, the adhesive molecule is preferably expressed by being activated.

In the kit for detecting carcinoma cells metastasizing to SLN, the adhesive molecule is preferably ICAM-1 or E-selectin.

In the kit for detecting carcinoma cells metastasizing to SLN, the adhesive molecule is preferably bonded with the carcinoma cells through a ligand.

In the kit for detecting carcinoma cells metastasizing to SLN, the ligand is preferably CD11a, CD11b and/or CD11c.

In the drug delivery agent for delivering the drug to the SLN comprises;
an antibody and/or a ligand therein which are exposed on the surface of colloid particles in the agent or suspended in the agent,
wherein the antibody and/or the ligand are interacted with an adhesive molecule mediated attachment of carcinoma cells metastasized from a primary tumor with LECs in SLN.

In the drug delivery agent, the antibody is preferably anti-ICAM-1 antibody and/or anti-E-selectin antibody.

In the drug delivery agent, the ligand is preferably a ligand of ICAM-1.

In the drug delivery agent, the ligand is preferably CD11a, CD11b and/or CD11c.

In the drug delivery agent, it is preferable that the colloid particles comprise and/or express a fluorescence agent, a contrast agent, a therapeutic agent and/or a potentiator for adhesion of the metastasized carcinoma cell.

In the drug delivery agent, the colloid particles are preferably micelle particles of a biodegradable resin, micelle particles of a synthetic resin or liposome.

The kit for detecting carcinoma cells metastasizing to SLN of the present invention can be used for simply and accurately detecting the carcinoma cells lymphogenously metastasizing from the primary tumor to the lymph node, in particular the SLN and, moreover, the detection of the carcinoma cells can be performed within a short period of time. In surgery of removing the primary tumor, the kit is useful for surely removing only the lymph node to which the malignant carcinoma cells are metastasized or the micro carcinoma cells are attached, and contributes to prevent cancer recurrence.

The drug delivery agent of the present invention preferentially reaches the SLN, to which the carcinoma cells are metastasized or the micro carcinoma cells are attached, and easily attaches to the carcinoma cells. Therefore, the drug delivery agent can be used to preferentially deliver the drug used for cancer diagnosis or medical cancer treatment to the carcinoma cells in the SLN.

### Brief Explanation of the Drawings

Fig. 1 shows time-dependent change in the expression of the adhesion molecules respectively stimulated by supernatants of the cultured carcinoma cell lines, which is detected by using the kit for detecting carcinoma cells metastasizing to SLN of the present invention.
Fig. 2 shows change in the expression of the adhesion molecules respectively stimulated by supernatants of cultured carcinoma cell lines, which is detected by using the kit for detecting carcinoma cells metastasizing to SLN of the present invention.
Fig. 3 shows change in the expression of the adhesion molecules stimulated by cytokines or growth factors, which is detected by using the kit for detecting carcinoma cells metastasizing to SLN of the present invention.
Fig. 4 shows effects of previously-treated supernatants of the cultured carcinoma cell lines on the expression of the adhesion molecules, which is detected by using the kit for detecting carcinoma cells metastasizing to SLN of the present invention.
Fig. 5 shows the effects treatment of ATP or suramin on the expression of the adhesion molecules, which is detected by using the kit for detecting carcinoma cells metastasizing to SLN of the present invention.
Fig. 6 shows the effects of treatment of suramin, 8-cyclopentyl-1,3-dipropylxanthine (DPCPX) or 3,7-dimethyl-1-propargyl xanthine (DMPX) on the expression of the adhesion molecules, which is detected by using the kit for detecting carcinoma cells metastasizing to SLN of the present invention.
Fig. 7 shows change in the adhesive capacity of the adhesion molecules by treatment with suramin, which is detected by using the kit for detecting carcinoma cells metastasizing to SLN of the present invention.
Fig. 8 shows change in the adhesive capacity of the adhesion molecules by treatment with anti-ICAM-1 antibody, which is detected by using the kit for detecting carcinoma cells metastasizing to SLN.
Fig. 9 shows staining on cultured cells by a lymph vessel marker.
Fig. 10 shows the effects of treatment of chemokines on the expression of the adhesion molecules, which is detected by using the kit for detecting carcinoma cells metastasizing to SLN of the present invention.
Fig. 11 shows the effects of stimulation time on the CCL2-mediated expression of the adhesion molecules, which is detected by using the kit for detecting carcinoma cells metastasizing to SLN of the present invention.
Fig. 12 shows the effects of CCL2 concentration on the expression of the adhesion molecules, which is detected by using the kit for detecting carcinoma cells metastasizing to SLN of the present invention.
Fig. 13 shows the effects of CCL2 neutralization on the CCL2-mediated expression of the adhesion molecules, which is detected by using the kit for detecting carcinoma cells metastasizing to SLN of the present invention.
Fig. 14 shows the effects of ICAM-1 antiserum on acceleration of the CCL2-mediated attachment of carcinoma cells on human LECs, which is detected by using the kit for detecting carcinoma cells metastasizing to SLN of the present invention.
Fig. 15 shows the results of the investigation on the expressions of CD11a and CD11b on carcinoma cells, which is detected by using the kit for detecting carcinoma cells metastasizing to SLN of the present invention.
Fig. 16 shows the results of the investigation on the expressions of E-selectin and ICAM-1 in the absence or presence of metastasis of the carcinoma cells to tissue of SLN.

### Detailed Explanation of the Invention

Hereunder, preferred embodiments of the present invention are explained in detail. However, the scope of the invention is not intended to be limited to those embodiments.

In the kit for detecting carcinoma cells metastasizing to SLN of the present invention, the human LECs is applied to the medium by dissemination etc., wherein the human LECs are collected through abrasion by intraluminal circulation of a protease solution such as a trypsin solution and a collagenase solution in lymph vessels, in particular extirpated human lymph vessels.

The lymph vessels are preferably human collecting lymph vessels, in particular afferent lymph vessels of human axillary lymph node. The human LECs are preferably cultured under low-oxygen atmospheric conditions after harvesting the endothelial cells. In the low-oxygen atmospheric conditions, the oxygen concentration is preferably 1 to 10%, more preferably 3 to 7%, still more preferably 5%. Because the oxygen concentration in lymph fluid in a human living organism is significantly low compared to the oxygen concentration in the blood therein, the culture condition of the low-oxygen atmospheric condition is considered to be the most suitable for the culture of the human LECs.

As an example of the collagenase solution used for abrasion of the LECs derived from human collecting lymph vessels, Collagenase Type II of Catalog No. S2B5456 (available from Worthington Biochemical Corporation, US), can be exemplified. The concentration thereof is preferably 0.01 to 0.1%, more preferably 0.05%. The intraluminal circulation speed of Collagenase Type II solution in the human collecting lymph vessel is not limited as long as the enzymatic action of Collagenase Type II can be expressed. The circulation may be interrupted to perform abrasion of the endothelial cells, or the abrasion of the endothelial cells may be performed while performing circulation. The composition of Collagenase Type II solution is not limited as long as the concentration of Collagenase Type II is within the above range.

Similar to human umbilical vein endothelial cells (HUVEC) of Catalog No.CC-2517 (available from Takara Bio Inc., Japan) and human microvascular endothelial cells (HMVEC) of Catalog No.CC-2505 (available from Takara Bio Inc., Japan) collected from human subcutaneous tissues, the human LECs are not limited to be ones derived from a single cell, and they can be repeatedly subcultured 10 times.

The human LECs are preferably performed with treatment for mycoplasma removal. The mycoplasma removal is preferably carried out by adding a mycoplasma removal agent to the endothelial cells to perform negative conversion and then purifying thereof. As long as the mycoplasma removal can be performed, the substance or the usage of the mycoplasma removal agent is not intended to be limited. Furthermore, the concentration of the mycoplasma removal agent is not intended to be limited. The mycoplasma removal can be performed at any passage under the subculture, but it is preferably performed at the second to fifth passage, more preferably at the second passage. The mycoplasma removal may be performed by using the mycoplasma removal agent for the cultured cells in an appropriate concentration, for example 15 times diluted solution of Mynox (available from Minerva Biolabs GmbH, Germany) or MC-210 (available from Dainippon Pharmaceutical Co., Ltd., Japan) The mycoplasma removal may be performed in conjugation with other removal methodologies.

Before performing the mycoplasma removal, the existence or nonexistence of mycoplasma infection may be examined. Before the mycoplasma removal, it can be examined at any passage under the subculture, but it is preferable to examine at the second to fifth passage, more preferably the second passage. The existence or nonexistence of mycoplasma infection may be examined by using a kit for detecting mycoplasma infection such as Mycoplasma Plus PCR Primer Set (available from Stratagene, US). As long as the existence or nonexistence of mycoplasma infection can be examined, the substance or the usage thereof is not intended to be limited. The examination of mycoplasma infection may be performed in conjugation with other removal methodologies.

The human LECs were received by Patent Organism Depositary, National Institute of Advanced Industrial Science and Technology having its address on 1-1, Higashi 1-chome Tsukuba-shi, Ibaraki-ken, Japan on January 18, 2006 and a domestic accession number of FERM P-20768 was given. And then the cells were entered for transfer to International Patent Organism Depositary of the same on January 16, 2009 and a reception number of FERM ABP-11089 was given.

For example, in the kit of the present invention, the endothelial cell line of the human LECs is plated on a growing substrate. The substrate may be any culture plate, culture slide glass or translucent film, and the shape and the modification condition on the culture surface thereof are not intended to be limited. The component composition of the culture medium used on the substrate of the kit is not intended to be limited as long as the cell lines can proliferate. For instance, EGM-2 of vascular endothelial cell culture medium (available from Sankyo Junyaku Co., Ltd., Japan) can be exemplified. Appropriate additives, for example cytokines such as hFGF, VEGF, R³-IGF-1, hEGF, VFGF-C, VEGF-D and PDGF-BB, various vitamins such as ascorbic acid, steroid such as hydrocortisone, and serum, can be added to the culture medium. The number of the human LECs on the substrate depends on the plated substrate area, but it's preferably 1x10⁴ to 1x10⁵ cells/cm².

In the kit for detecting carcinoma cells metastasizing to SLN, it is preferable that an adhesion molecule such as intercellular adhesion molecule (ICAM-1) or E-selectin, which mediates the adhesion of the carcinoma cells metastasizing to the human LECs applied on the substrate, is expressed by being activated. The adhesive molecule is further preferably ICAM-1.

In the kit for detecting carcinoma cells metastasizing to SLN, the adhesion molecule, which mediates the adhesion of the carcinoma cells metastasizing to the human LECs, is interacted with the carcinoma cells through a ligand in the adhesion molecule. As such ligand, a ligand of ICAM-1 such as CD11a, CD11b and CD11c can be exemplified.

It is further preferable that the kit for detecting carcinoma cells metastasizing to SLN further comprises an immunoassay detecting agent for immunostaining of the adhesive molecules.

The immunoassay detecting agent can be used for indirect immunohistochemical analysis using an antigen-antibody reaction. The immunoassay detecting agent is not intended to be limited as long as it can be used for labeling immunohistochemical analysis using a labeled antibody, for example immunonephelometry for optically detecting the precipitation or aggregation reaction, radioimmunoassay, enzyme immunoassay and fluorescent immunoassay.

The kit for detecting carcinoma cells metastasizing to SLN is used as follows. Lymph fluid, for example lymph fluid in the SLN collected from the breast cancer patient, is added on the substrate of the kit. When there are the metastatic carcinoma cells, for example micrometastatic carcinoma cells, metastasized from the primary tumor in the lymph fluid, the metastatic carcinoma cells attach to the human LECs through the adhesion molecules on the human LECs. When the adhesion molecules are detected by immunoassay, it can be confirmed that those carcinoma cells are naturally metastasized to the SLN because there are micrometastatic cells in the lymph fluid.

In the drug delivery agent, the antibody against the adhesive molecule, for example anti-ICAM-1 antibody and anti-E-selectin, which mediates the attachment of the carcinoma cells metastasized from the primary tumor to the LECs in the SLN, is exposed on the surface of colloid particles. In the drug delivery agent, a ligand which interacts with the adhesive molecule, for example CD11a, CD11b and/or CD11c, may be exposed on the surface of the colloid particles.

The commercially available anti-ICAM-1 antibody, anti-E-selection antibody and ICAM-1 ligand such as CD11a, CD11b and CD11c can be used.

Such ICAM-1 agonist expresses on the surface of the carcinoma cells in large numbers, and it was found that the ICAM-1 agonist is interacted with the adhesion molecules expressed on the human LECs. By using this mechanism, the drug delivery agent of the present invention can be used for delivering the drug in a site-specific manner. ICAM-1 expresses in the endothelial cells in the lymph node to which the carcinoma cells are metastasized to mediate attachment with the endothelial cells.

Examples of the colloid particles are micelle particles of biodegradable resin, micelle particles of synthetic resin and liposome particles. It is preferable that these particles are nanoparticles having the average particle size of 5nm to 500nm. If the particle size is less than 5nm, the particles are immediately excreted from a living organism. If the particle size is more than 500nm, the particles are removed from the living organism as a foreign substance. When the particle size is approximately 200nm, the particles are easily absorbed especially into interstitium between cells in vascular injury regions or smooth muscle cells exposed intravascularly. The drug delivery agent preferably comprises 0.5 to 2.0% of the colloid particles. Suspended polylactic acid particles can be exemplified as the biodegradable resin particles. As the synthetic resin particles, polystyrene beads having the average particle size of 200nm can be exemplified. As example of the liposome, liposome made from fat or phospholipid having a diameter of 50 to 800nm, more preferably 200 to 400nm.

In the drug delivery agent, it is preferable that the medicinal substance such as a fluorescence agent, a contract agent, a therapeutic agent and/or a potentiator for adhesion of the metastasized carcinoma cells is included in the colloid particles or is attached or bonded to the colloid particles to be exposed.

In the drug delivery agent, the medicinal substance and the before-mentioned antibody or the ligand may be bonded or interacted with being suspended. For example, the drug delivery agent comprises the colloid particles including the ligand of ICAM-1 such as CD11a, CD11b and CD11c, to which the medicinal substance of the fluorescent agent is bonded. Thus drug delivery agent is used for fluorescence microscope observation by in vitro attachment to the cells. The agent is also used to in vivo delivery of the drug to the desired biological region. As the fluorescent agent, a fluoroscein isothiocyanate (FITC), Calcein-AM of viable cell staining dye (available from Dojindo Laboratories, Japan) can be exemplified. Also, the drug delivery agent may comprise the colloid particles including the chemokine to which an infrared-chromogenic dye of indocyanine green is bonded.

As the contrast agent, a gadolinium compound for magnetic resonance for diagnostic imaging and an iodine compound for X-ray tomography can be exemplified. As the therapeutic agent, a vascular endothelial cell growth accelerator, a vascular smooth muscle cell growth inhibitor, an antiinflammatory agent and an anticancer agent can be exemplified while it can be a potentiator for adhesion of the metastasis carcinoma cells.

The drug delivery agent is used as follows. The drug delivery agent is injected to a primary tumor or a nearby lymph node, and then the drug delivery agent is delivered to SLN by a lymphatic circulation. If the carcinoma cells, for example micro-carcinoma cells, are metastasized to the endothelial cells of either the lymph vessel or SLN which lie downstream of the primary tumor, the adhesion molecule which mediates the adhesion of the carcinoma cells, for example ICAM-1, are expressed on the endothelial cells. The antibody against the adhesion molecule in the drug delivery agent performs an antigen-antibody reaction to be bonded with the adhesion molecule. Also the ligand of the adhesion molecule in the drug delivery agent performs an enzymatic reaction to be selectively bonded with the adhesion molecule as if it is a lock-and-key model. And then the fluorescence agent, the contrast agent, the therapeutic agent or the potentiator for adhesion of the metastasized carcinoma cell may exude to be released from the surface of the colloid particles in the drug delivery agent or may be absorbed to the metastasized carcinoma cells, and therefore produces fluorescence or contrast-imaging, or expresses medicinal benefits.

For example, by using ultrasonography, magnetic resonance imaging (MIR) or X-ray tomography (CT), the location of the SLN with the metastasized carcinoma cells to which the drug delivery agent is preferentially delivered can be visualized by photographic images. Even one or two micro-metastatic carcinoma cells attached to the SLN build microenvironment in the lymph node suitable for micro-metastasis and express the adhesion molecules. Therefore, the adhesion molecules can be accurately detected and the lymph node to be removed can be accurately and promptly specified. As the drug delivery agent, a contrast agent for MRI including metal such as gadolinium wherein the adhesion molecule antibody or the ligand of the adhesion molecule is exposed thereon can be exemplified.

The drug delivery agent can be used for increasing the adhesion of the micro-metastatic cells captured by the SLN to prevent the cells from metastasizing to the lymph node located downstream. Moreover, because the drug delivery agent has a controlled-release property, the medicinal benefits of the drug can be maintained over time.

Hereunder, embodiments of the kit for detecting carcinoma cells metastasizing to SLN and the drug delivery agent of the present invention are explained in detail.

Reagents and methods used for the Examples are as follows.

### (1. Cell Culture)

Isolation and culture of human LECs were performed using techniques of Kawai et al. (Kawai et al., Lymphatic Research and Biology, 2007, vol.5, pp. 115-126 and 2008, vol.6, pp. 15-27) with nearest afferent lymph vessels of SLN in patients with breast cancer. Experimental protocols were approved by the ethical committee for human studies in the School of Medicine, Shinshu University. The patients were informed of the risks and purposes of the studies before their written consents were obtained.

### (1.1 Preparation of human lymphatic endothelial cells (human LECs))

Afferent lymph vessels of SLN were extirpated from the breast cancer patients, who assented and signed the written consents before the operation, by breast endocrine surgery operations with biopsies of SLN in Department of Breast & Endocrine Surgery in the School of Medicine, Shinshu University. The dissected lymph vessel of the SLN was cannulated centripetally with a sterile polyethylene tube and intraluminally circulated for 10 min. with pre-warmed (37°C) 500U/mL trypsin/ethylenediamine tetraacetic acid solution. After enzymatic digestion, intraluminal fluid containing endothelial cells was gently drained into a centrifuge tube with endothelial growth medium (EGM)-2 (available from Clonetics, US) and 10% fetal bovine serum (FBS). The collected solution was centrifuged at 2,000 r.p.m. for 5 min. at 4°C. The supernatants were removed, and the pellets were resuspended in EGM-2 culture medium, and then plated on a 35 mm culture plate (available from Corning, US) coated with type I collagen (available from Nitta Gelatin, Japan). Human LECs from the afferent lymph vessels of the SLN of the breast cancer patients were maintained in EGM-2 with 10% FBS and used at the fifth to seventh passage. The LECs were incubated under atmospheric conditions of 5% O₂, 5% CO₂, and 90% N₂ at 37°C.

The isolation and culture of human collecting lymphatic endothelial cell line were performed using another technique as follows. Human collecting lymph vessels as afferent lymph vessels of human axillary lymph node were extirpated from patients with breast cancer who also assented and signed on a written consent, with surrounding tissue by surgery. To prevent contamination of isolated cells from the human collecting lymph vessel by other extraneous substance, the tissues around the lymph vessels such as fat and capillary vessels were decorticated under stereomicroscopic observation. To perform circulation, washing and harvest of the human LECs, a narrow polyethylene tube was intraluminally cannulated to the human collecting lymph vessels and then indwelt. The vessels were washed with phosphate buffered saline (PBS solution) by intraluminal circulation and then intraluminally loaded by 0.05% of Collagenase Type II of Catalog No.S2B5456 (available from Worthington Biochemical Corporation, US). They were gently incubated at 37°C for appropriate time until abrasion of endothelial cells was observed in an incubator, approximately for 10 min. As culture medium and its additives, 500 mL of EGM-2 BulletKit of vascular endothelial cell growth medium for proliferation of catalog No.CC-3162 (available from Sankyo Junyaku Co., Ltd., Japan) and 40 mL of Fetal Bovin Serum (FBS) of catalog No.S1560 (available from Japan BioSerum, Japan) were used. The human collecting lymph vessel was intraluminally circulated with EGM-2 containing 10% FBS, and then the endothelial cells, which were intraluminally abraded, were collected. The collected liquor of the endothelial cells was poured in a tube, and the tube was centrifuged at 2000 r.p.m. for 5 min. to separate the endothelial cells. The human LECs derived from the collecting lymph vessels were isolated. 1x10⁵ cells of the endothelial cells from the isolated LECs were plated on a cell culture plate having 35mm thickness coated with type I collagen of Catalog No. I-P (available from Nitta Gelatin, Japan) for improvement of the cells, and then 1.5 mL of EGM-2 containing 10% FBS as culture medium was added. The cells were incubated under low-oxygen atmospheric conditions of 5% O₂, 5% CO₂, and 90% N₂ at 37°C. Doubling time thereof was approximately 48 hrs. The cells were incubated to confluence, washed with PBS solution, and treated with 0.25% of trypsin solution at 37°C for 3 min. The cells were centrifuged for 5 min. at 2000 r.p.m. to be harvested and then plated to 3 or 4 piece of flesh cell culture plates having 35mm thickness coated with type I collagen. The cells were incubated to confluence and then subcultured properly. Because these cells were derived from the patients, the cells might already be infected with mycoplasma on primary subculture passage. Therefore the existence or nonexistence of mycoplasma infection of the cells on second subculture passage was examined by using a kit for detecting mycoplasma infection: Mycoplasma Plus PCR Primer Set (available from Stratagene, US). When the result of the examination for mycoplasma infection was positive, mycoplasma removal was performed by using a mycoplasma removal agent for the cultured cells of at least one of 15 times diluted solution of Mynox (available from Minerva Biolabs GmbH, Germany) and 0.5µg/mL of MC-210 (available from Dainippon Pharmaceutical Co., Ltd., Japan) After being negative conversion of the mycoplasma, circumstances of mycoplasma infection of the cells were checked by the kit for detecting mycoplasma infection under appropriate subculture passages, for example, by approximate two subculture passages, and the cell line being negative mycoplasma infection was established.

### (1.2 Evaluation of proliferative ability for human LECs)

The human LECs were respectively incubated under a low-oxygen atmospheric condition of 5% oxygen and a normal oxygen condition of 20% oxygen for 96 hrs. After the incubation, cell number per field of view field was counted by microscopic observation to investigate the difference of proliferative ability of the human LECs between the cases in low-oxygen concentration and normal-oxygen concentration. As a result, the LECs cultured under the low-oxygen condition have superior proliferative ability than LECs cultured under the normal-oxygen condition.

### (1.3 Stock of human LECs)

Procedures to stock the human LECs are as follows. The endothelial cell line was harvested with 0.25% trypsin solution, and then suspension of the cells was prepared with a preservative agent for freezing consisting 10% of dimethylsulfoxide (DMSO) and 90% of FBS to be poured into freezing tubes. The tubes were cooled down stepwise in a freezing vessel: Bicell, and then frozen with liquid nitrogen to be stocked. Procedures for thawing and culturing thereof are as follows. The freezing tubes were warmed up in a thermostat bath at 37°C to thaw the cells. The tubes were centrifuged at 2000 r.p.m. for 5 min., and the endothelial cells therein were harvested and suspended in EGM-2 containing 10% FBS as cell culture medium to prepare a cell suspension. Then the suspension was incubated as same as previously mentioned procedures of culture for the endothelial cells.

### (1.4 Biological properties of human LECs)

### (1.4 A. Observation of immunostaining through CD31 (platelet endothelial cell adhesion molecule: PECAM))

LECs derived from the human collecting lymph vessel, which were transplanted into a culture system, were plated on glass slide, repeatedly subcultured to confluence, and then fixed with 10% formalin. After they were blocked with PBS solution containing 0.1% bovine serum albumin (BSA), a primary antibody: CD31 of Catalog No.sc-8306 (available from Santa Cruz, US) as an endothelial cell marker diluted 100 times with PBS solution containing 0.1% BSA was added thereto. They were still stood overnight at 4°C. After the primary antibody was washed with PBS solution, fluorescent-labeled goat anti-rabbit immunoglobulin G-FITC of Catalog No. sc-2012 (available from Santa Cruz, US) as a secondary antibody against the primary antibody diluted 100 times with PBS solution containing 0.1% BSA was added thereto. They were still stood for 1 hr. at room temperature.

After the secondary antibody was washed out with PBS solution, they were mounted in Mobi GLOW Mounting Medium of Catalog No. MGM01 (available from MoBiTec, Germany) to be observed by a fluorescent microscope. The LECs indicate green fluorescence, therefore they are positive against CD31.

### (1.4 B. Observation of immunostaining through VEGF-R3 (vascular endothelial growth factor receptor 3))

LECs derived from the human collecting lymph vessel, which were transplanted into a culture system, were plated on glass slide, repeatedly subcultured to confluence, and then fixed with 10% formalin. After they were blocked with PBS solution containing 0.1% bovine serum albumin (BSA), a primary antibody: VEGF-R3 of Catalog No. sc-637 (available from Santa Cruz, US) as a specific lymphatic endothelial cell marker diluted 100 times with PBS solution containing 0.1% BSA was added thereto. They were still stood overnight at 4°C. After the primary antibody was washed out with PBS solution, fluorescent-labeled goat anti-rabbit immunoglobulin G-FITC of Catalog No. sc-2012 (available from Santa Cruz, US) as a secondary antibody against the primary antibody diluted 100 times with PBS solution containing 0.1% BSA was added thereto. They were still stood for 1 hr. at room temperature. After the secondary antibody was washed out with PBS solution, they were mounted in Mobi GLOW Mounting Medium of Catalog No. MGM01 (available from MoBiTec, Germany) to be observed by a fluorescent microscope. The LECs indicate green fluorescence, therefore they are positive against VEGF-R3.

### (1.4 C. Observation of immunostaining through LYVE-1 (lymphatic vessel endothelial hyaluronan receptor-1))

The LECs were observed as same as above-mentioned case of immunostaining through VEGF-R3 except for using another specific lymphatic endothelial cell marker: LYVE-1 of Catalog No. sc-19316 (available from Santa Cruz, US) as a primary antibody and fluorescent-labeled donkey anti-goat immunoglobulin G-FITC of Catalog No. sc-2024 (available from Santa Cruz, US) as a secondary antibody. The LECs indicate green fluorescence, therefore they are positive against LYVE-1.

As evident results of observation of immunostaining through CD31, VEGF-R3 and LYVE-1, the LECs maintained biological properties of the endothelial cells due to expression of CD31, VEGF-R3 and LYVE-1, even if the LECs were transplanted into a culture system. Therefore it was obvious that the isolation and culture of the LECs line were established.

### (1.4 D. Observation of staining through cytoskeletal protein: F-actin)

LECs derived from human collecting lymph vessel, which were transplanted into a culture system, were plated on glass slide, and repeatedly subcultured to confluence. Stimulating factors of 10ng/mL of tumor necrosis factor-α (TNF-α) of Catalog No. T-0157 (available from SIGMA, US), 1ng/mL and 10ng/mL of interleukin-1β (IL-1β) of Catalog No. 200-01B (available from PeproTech, US) were respectively dissolved in EBM-2 containing 3% FBS of Catalog No. CC-3156 (available from Sankyo Junyaku Co., Ltd., Japan) and added onto the cells, and then the cells were incubated for 2 hrs. at 37°C. They were fixed with 3.7% formalin. They were washed with PBS solution, and then still stood in PBS solution containing 0.1% Triton X-100 of Catalog No. X-100 (available from SIGMA, US) for 5 min. Additionally they were washed with PBS solution, and then phalloidin-FITC antibody of Catalog No. P-5282(available from SIGMA, US) diluted 500 times with PBS solution containing 0.1% BSA was added thereto. They were still stood for 2 hrs. at room temperature. After they were washed with PBS solution, and mounted in Mobi GLOW Mounting Medium. Also negative control was similarly prepared except for using no stimulating factor. Those were observed by a fluorescent microscope to be compared each other. The LECs of the negative control without the stimulating factor indicate obscure green fluorescence, and there were few expression of F-actin. While the LECs stimulated with TNF-α or IL-1β indicate bright green fluorescence, and increase of expression of F-actin was observed.

### (1.5 Otherwise characteristics of Morphology, culture, and physiology of human LECs)

### (1.5.1. Morphometric characteristics)

### (1) Morphology and Size

The LECs have respectively polygonal shape which cultured endothelial cells have as a fundamental property, and indicate cobblestone appearance in monolayer. Size thereof is approximately 50 µm in diameter.

### (2) Polymorphism

The LECs indicate almost similar morphological shape, but do not indicate polymorphism.

### (1.5.2. Culture characteristics)

### (1) Culture medium

The culture mediums were prepared by adding Fetal Bovine Serum (FBS) to EGM-2 as a medium for proliferating the vascular endothelial cells to be 10% of final FBS concentration.

### (2) Culture conditions

The LECs were incubated under atmospheric condition of 5% O₂, 5% CO₂, and 90% N₂ at 37°C in an incubator.

### (3) Physiological characteristics

### (3.1) Expression of LECs marker

Expressions of an endothelial cell marker of CD31 and lymphatic endothelial cell markers of Prox-1, LYVE-1 and podoplanin were observed in the LECs.

### (3.2) Physiological features

Doubling time of the LECs was 48 hrs. Sthenia of proliferative ability of the LECs by lymphangiogenic factors such as basic-FGF, VEGF and VEGF-C was observed.

### (1.6 Preparation of human breast cancer cell lines)

The human breast adenocarcinoma cell lines: MDA-MB-231 of high-metastatic clone and MCF-7 of low-metastatic clone, were purchased from American Type Culture Collection (US). The carcinoma cells maintained in Dulbecco's modified Eagle's medium/Nutrient Mixture F12 Ham (DMEM/F12) culture medium supplemented with 10% FBS. The LECs were incubated under atmospheric conditions of 5% O₂, 5% of CO₂ and 90% N₂ at 37°C, whereas the carcinoma cells were incubated under normal conditions of 21% O₂, 5% of CO₂ and 74% N₂ at 37°C.

### (2. Measurment and Observations)

### (2.1 Cytokine and growth factor assays)

The concentrations of cytokines and growth factors in the supernatant of the culture medium of MDA-MB-231 or MCF-7 were determined by Enzyme-Linked Immuno Sorbent Assay (ELISA), specific against human cytokines and growth factors. When the supernatants were collected, the carcinoma cells were plated in DMEM/F12 with 10% FBS, which was replaced the following day with DMEM/F12 with 0% FBS and collected after overnight culture. The collected solution was centrifuged at 2,000 r.p.m. for 5 min. at 4°C and then kept in frozen at -20°C for assays of cytokines or growth factors. Levels of tumor necrosis factor-α (TNF-α), tumor growth factor-β1 (TGF-β1), interferon-γ (INF-γ), interleukin-1β (IL-1β), IL-6, IL-12, basic fibroblast growth factor (bFGF), platelet derived growth factor-BB (PDGF-BB), vascular endothelial growth factor-A (VEGF-A), and VEGF-C were measured commercially (SRL, Tokyo, Japan). Detection limits were 5pg/mL for TNF-α, 0.5ng/mL for TGF-β1, 0.1U/mL for INF-γ, 10pg/mL for IL-1β, 0.2pg/mL for IL-6, 7.8pg/mL for IL-12, 10pg/mL for basic FGF, 31.2pg/mL for PDGF-BB, 20pg/mL for VEGF-A, and 109pg/mL for VEGF-C.

### (2.2 ATP Assay)

The concentrations of ATP in the supernatant of culture medium of MDA-MB-231 or the culture medium (DMEM/F12) in the absence or presence of ATP (10⁻⁸M, 10⁻⁷M and 10⁻⁶M) were determined using the luciferin-luciferase assay based on Cell Titer-Glo Luminescent Cell Viability Assay (Cell Titer-Glo is a registered trademark; Promega Corporation, US). Thus, 100µL of the MDA-MB-231 supernatant or 100µL of the culture medium in the absence or presence of ATP was collected into a 96-well plate, to which 100µL of luciferine-luciferase solution was added, and light emission was recorded by a luminometer (available from Dainippon Sumitomo Pharma, Japan).

### (2.3 (A) Immunohistochemical studies (I))

Using an indirect immunohistochemical technique, the inventors examined the effects of the supernatants of culture medium of two kinds of carcinoma cells, MDA-MB-231 and MCF-7, on the expression of adhesion molecules such as E-selectin, P-selectin, vascular cell adhesion molecule (VCAM)-1, and intercellular adhesion molecule (ICAM)-1 on the human LECs. The carcinoma cells were plated in DMEM/F12 with 10% FBS, which was replaced the following day with DMEM/F12 with 3% FBS, and then collected after overnight culture. The collected solution was centrifuged at 2,000 r.p.m. for 5 min. at 4°C. To examine the effects of the supernatants on the expression of the adhesion molecules on the human LECs, 1mL of each collected solution was added to starvation culture medium, EBM-2 with 3% FBS of the human LECs, and then stimulated for 48 hrs. on the cells. In some experiments, the supernatant solutions at different concentrations (1/100 or 1/10,000 dilution) were constructed with appropriate volumes of DMEM/F12 with 3% FBS.

Indirect immunohistochemical studies were performed on the cultured LECs seeded or glass slides coated with type I collagen, and then the cells were fixed with 3.3% formalin in phosphate-buffered saline solution (PBS), for 20 min. at room temperature. The cells were washed three times with PBS, and then incubated overnight at 4°C with primary polyclonal human antisera to E-selectin/CD62E (dilution 10µ/mL, available from R&D Systems, US), P-selectin/CD62P (dilution 10µg/mL, available from R&D Systems, US), VCAM-1/CD106 (dilution 10µg/mL, available from R&D Systems, US), and ICAM-1/CD54 (dilution 10µg/mL, available from R&D Systems, US).

Before primary staining, the cultured cells were permeabilized with 0.1% Triton-X. After washing three times in PBS, the cells were incubated for 1 hr. at room temperature with 1:100 diluted Alexa Fluor 488 donkey anti-mouse IgG (available from Invitrogen, US). The nuclei of cultured cells were counter-stained and mounted with ProLong Gold antifade reagent with 4'-6-diamidine-2-phenylindole (DAPI) (available from Molecular Probes, US), examined by a fluorescent microscope (Leica, Switzerland), and photographed.

For non-specific staining, Block-Ace (available from Dainippon Sumitomo Pharma Co., Ltd., Japan) was substituted for primary antisera as a negative control.

### (2.3 (B) Immunohistochemistry (II))

Immunohistochemistry was performed on the cultured human LECs or the fresh-frozen SLNs isolated from the breast cancer patients. In brief, the cultured LECs were fixed with 10% formalin in the phosphate-buffered saline solution (PBS) at room temperature. The cells were incubated for 4 hrs. at room temperature with primary polyclonal human antisera platelet-endothelial cell adhesion molecule (PECAM) -1 (dilution 1:100, available from BD Biosciences, US), lymphatic vessel endothelial hyaluronan receptor (LYVE) -1 (dilution 1:50, available from RELIATech, Germany), Prox-1 (dilution 1:50, available from AngioBio, US), podoplanin (dilution 1:50, available from AngioBio, US), vascular endothelium growth factor receptor 3 (VEGF R3) (dilution 1:50, available from Santa Cruz, US), E-selectin / CD62E (dilution 1:50, available from R&D systems, US), P-selectin / CD62P (dilution 1:50, available from R&D systems, US), vascular cell adhesion molecule (VCAM) -1 /CD106 (dilution 1:50, available from R&D systems, US), and intercellular adhesion molecule (ICAM) -1 / CD54 (dilution 1:50, available from R&D systems, US). Before the staining, permeabilization of the cultured cells with 0.1% Triton-X was performed. Then, the cells were stained using the following antibodies: Alexa Fluor 488 chicken anti-rabbit IgG or Alexa Fluor 488 donkey anti-mouse IgG (available from Invitrogen, US). The nuclei of the cultured cells were counter-stained and mounted with ProLong Gold antifade reagent with 4'-6-diamidine-2-phenylindole (DAPI) (available from Molecular Probes, US). The cultured cells were examined by a fluorescent microscope (available from Leica, Germany) and photographed.

On the other hand, the fresh-frozen SLNs tissues were fixed with 100% acetone at 4°C. Endogenous peroxidase activity was blocked with 0.3% H₂O₂ for 30 min. at room temperature. The tissues were incubated for 1 hr. at room temperature with primary polyclonal antisera E-selectin (available from R&D systems, US) and ICAM-1 (available from R&D systems, US) and then for 30 min. at room temperature with horseradish peroxidase-labeled anti-rabbit IgG and anti-mouse IgG (available from Nichirei, Japan). The reaction product was developed using the DAB kit (available from Nichirei, Japan). The nuclei of the SLN tissues were also counter-stained using hematoxylin staining. The SLN tissues were examined by a light microscope (available from Leica, Germany) and photographed.

To examine the effects of chemokines on the immunohistochemical expression of the adhesion molecules on the LECs, the starvation culture medium was exchanged for 1mL of EBM-2 with 3% FBS contained various concentrations of chemokines and then stimulated the human LECs for 4 hrs., 18 hrs., or 48 hrs. The various concentrations of chemokines were constructed by diluting each chemokine with appropriate volumes of EBM-2 with 3% FBS.

In some experiments, the effects of 10ng/mL CCL2 on the immunohistochemical expression of ICAM-1 on the human LECs were also evaluated using the same procedure as that mentioned above after overnight neutralization of CCL2 in the starvation culture medium with a CCL2 specific antibody (1.0µg/mL).

To obtain positive controls of primary antisera to E-selectin, P-selectin, VCAM-1, and ICAM-1, the effects of 18 hrs. stimulation of 10ng/mL tumor necrosis factor (TNF)-α or 100ng/mL lipopolysaccharide (LPS) on the immunohistochemical expression of the adhesion molecules on the human LECs were examined.

For non-specific staining, Block-Ace (available from Dainippon Sumitomo Pharma, Japan) was substituted for primary antisera as a negative control.

To quantitatively examine the immunohistochemical data concerning the expression of ICAM-1 on the human LECs, the high-resolution digital microphotographs were processed using the Scion Image analysis program. The constant area of each LECs was outlined on the gray scale image and processed for density measurement. The results were expressed in arbitrary units (mean density / pixel). The data are shown as mean ± S.E.M (n=5).

### (2.4 In vitro human LECs attachment assay)

The human LECs were plated to form a monolayer on type I collagen-coated 35mm plates and incubated to confluence in 5% O₂, 5% CO₂, and 90% N₂ at 37°C. The LECs were kept in serum-starved medium containing EBM-2 with 3% FBS. Selected plates were treated with 10ng/mL CCL2 for 18hrs. In some experiments, the plates were stimulated by the serum-starved medium pretreated with neutralization of 10ng/mL CCL2 with 1.0µg/mL CCL2 specific antibody.

In some experiments, the plates were also treated with anti-human ICAM-1 antibody (available from R&D systems, US) for 30 min. after the 18hrs. treatment with 10ng/mL CCL2.

The two kinds of the breast carcinoma cells such as MDA-MB-231 and MCF-7 stained with PKH26 fluorescent dye (available from SIGMA, US) were then plated at 1x10⁵ cells per plate and incubated for 30 min. at 37°C. Unbound cells were removed by aspiration, and the plates were washed with EBM-2 three times. The attachment of the carcinoma cells was quantified by counting the number of cells under x100 magnification using a Leica microscope.

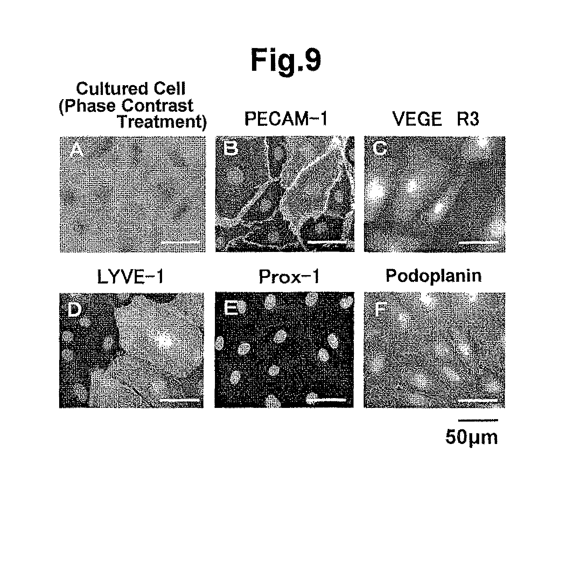

To analyze immunohistochemical expression of counter receptors / ligands against ICAM-1 on the breast carcinoma cells, the expressions of LFA-1 (CD11a) and Mac-1 (CD11b) on MDA-MB-231 and MCF-7 breast carcinoma cell lines were evaluated. The immunohistochemical procedure is quite the same as that adopted in the studies of the lymph vessel markers (Fig. 9). In the experiment, polyclonal antisera to CD11a (dilution 1:50, available from AnaSpec, US) and CD1lb (dilution 1:50, available from R&D systems, US) were used.

### (2.5 Quantitative RT-PCR)

The 0 hr., 1 hr., 4 hrs. and 18 hrs. expressions of ICAM-1 mRNA were evaluated by quantitative reverse transcription polymerase chain reaction (RT-PCR) for ICAM-1 cDNA. The total RNA was extracted from the cultured human LECs using Isogen reagent (available from Nippon Gene Co., Ltd., Japan) according to the manufacturer's instructions. The concentration of each RNA was calculated using 260nm absorbance with a spectrophotometer. The extracted RNA was reverse-transcribed with M-MLV reverse transcriptase (available from Ambion Inc., US). For RT-PCR analysis, each superscript first-strand synthesis kit (available from Invitrogen Corporation, US) was used with 1.0µg of the total RNA. Forward and reverse primers of ICAM-1 and cyclophilin A were used for each specific probe, respectively as follows; ICAM-1 (available from TAKARA BIO INC., Japan), and cyclophilin A; 5'-TTCGTGCTCTGAGCACTGGAG-3' (forward; Sequence No.1 in Sequence Listing) and 5'-GGACCCGTATGCTTTAGGATGAAG-3' (reverse; Sequence No.2 in Sequence Listing). The cDNA was diluted 5-fold prior to PCR amplification. Quantitative RT-PCR was performed using a Light Cycler rapid thermal cycler system (available from Roche Diagnostics Limited, UK). Reactions were performed in a 20µL volume with 0.5µM primers, Taq DNA polymerase, and the buffer was included in the SYBR Premix Ex Taq (available from TAKARA BIO INC., Japan; SYBR is a registered trademark). The PCR protocol included a 10 secs. of denaturation step followed by 45cycles of 95°C denaturation for 5 secs. and 60°C annealing for 20 secs. The fluorescent product was detected at the end of the 72°C extension period. Negative controls included PCR reactions with cDNA omitted. To confirm amplification specificity, PCR products from each primer pair were subjected to melting curve analysis. Quantification data was analyzed with Light Cycler analysis software. The results are presented as normalized ratio of the expression of ICAM-1 mRNA to cyclophilin A.

### (2.6 Western Blot Analysis)

Western blot analysis was performed to quantitatively evaluate the CCL2-mediated ICAM-1 expression on the cultured human LECs. The cells were dissolved in M-PER Mammalian Protein Extraction Reagent (available from Thermo Fisher Scientific Inc., US) and centrifuged at 14,000 r.p.m. for 10 min. A 15µg sample of the total cell lysate was resolved in SDS sample buffer for sodium dodecyl sulfate-polyacrylamide gel electrophoresis (SDS-PAGE) and transferred to an polyvinylidene difluoride (PVDF) membrane (available from Atto Corporation, Japan), where it was incubated for 45 min. The membrane was probed with the anti-ICAM-1 antiserum (dilution 1:1000, available from Cell Signaling Technology Inc., US) and then incubated with anti-rabbit immunoglobulin G (IgG) horseradish peroxidase conjugated antibody. The same membrane was reprobed with monoclonal anti-actin antibody (available from Santa Cruz Biotechnology Inc., US) and then visualized with an ECL-Western blotting detection system (available from Amersham Bioscience Inc., UK).

### (3 Preparation of kit for detecting carcinoma cells metastasizing into sentinel lymph node and drug delivery agent, and evaluation thereof)

### (3.1 Preparation)

To evaluate the chemical properties of excitatory substances released from MDA-MB-231 cells, the effects of chemically or physically modified supernatants on the immunohistochemical expression of ICAM-1 on human lymphatic endothelial cells, with 48 hrs. of treatment, were studied. The supernatant was heated at 80°C for 30 min., and then treated with protease (pronase E, 1µg/mL, available from SIGMA, US) at 37°C overnight, the reaction of which was terminated by heating at 80°C for 30 min. Finally, the supernatants were dialyzed using two kinds of tubing of the dialysis membrane (mol wt 1,000 or 500, available from Spectum Medical Industries, US). The tubing was put into a buffer medium (DMEM-F12 (1:1) medium) for dialysis at 4°C overnight. The supernatant trapped inside the membrane was then used for the bioassay. Thus, the supernatant contained no chemical substance <1,000 or <500 in molecular weight.

To evaluate the pharmacological properties of the excitatory substances released from MDA-MB-231, the effects of the MDA-MB-231 supernatant and ATP (10⁻⁸ and 10⁻⁷M) on the expression of ICAM-1 at 48 hrs. on the human lymphatic endothelial cells were investigated in the absence or presence of suramin (10⁻⁷ and 10⁻⁶M, an antagonist of P2X and P2Y receptors, or 8-cyclopentyl-1,3-dipropylxanthine (10⁻⁷ and 10⁻⁶M, DPCPX, a selective adenosine A₁ antagonist), or 3,7-dimethyl-1-proparly xanthine (10⁻⁷ and 10⁻⁶M, DMPX, a selective adenosine antagonist).

To examine quantitatively the data of the immunohistochemical expression of ICAM-1 on the human LECs, high-resolution digital photomicrographs were processed using the Scion Image analysis program. Five constant areas of each LEC were outlined on the grayscale image and processed for density measurement. The results are expressed in arbitrary units (mean density/pixel).

### (3.2 In vitro human LEC attachment assays)

The human LECs were plated to form a monolayer on type I collagen-coated 35mm plates and incubated to confluence at 37°C in 5% O₂, 5% CO₂, and 90% N₂. The LECs were kept in serum-starved medium of EBM-2 with 3% FBS. Selected plates were treated with 10⁻⁷M ATP or the supernatant of culture medium of MDA-MB-231 cells for 48 hrs. In some experiments, 10⁻⁶M suramin was simultaneously added to the plates during 48 hrs. treatment with 10⁻⁷M ATP or the culture medium supernatant of MDA-MB-231 cells.

In some experiments, the plates were also treated with anti-human-ICAM-1 antibody (available from R&D Systems, US) for 30 min. after 48 hrs. treatment with the culture medium supernatant of MDA-MB-231 or 10⁻⁷M ATP. Breast carcinoma cells stained with PKH26 fluorescent dye (available from SIGMA, US) were then plated at 5 x 10⁴ cells per plate and incubated for 30 min. at 37°C. Unbound cells were removed by aspiration and the plates washed with DMEM/F12 three times. Attachment was quantitated by counting cells under x100 magnification using a microscope (available from Leica, Switzerland).

### (4.1 Reagent)

All salts were obtained from Wako (Tokyo, Japan): ATP and suramin from SIGMA, US; DPCPX and DMPX from Research Biologicals. DPCPX was diluted with ethanol and DMPX was diluted with dimethyl sulfoxide (DMSO). The concentrations of ethanol and DMSO did not affect the biological viability of the culture cells. Reagent concentration was expressed as the final concentration in the culture plate.

### (4.2 Statistical analysis)

All results are expressed as the mean ± standard error of the mean (SEM), and statistical significance was analyzed by Student's t-test for unpaired observations. p<0.05 was considered significant. P<0.01 was considered sufficiently significant.

### (5. Results of evaluation of kit for detecting carcinoma cells metastasizing into sentinel lymph node and drug delivery agent)

### (5.1 Effect of MDA-MB-231 supernatant or MCF-7 concerning expression of adhesion molecules on LECs)

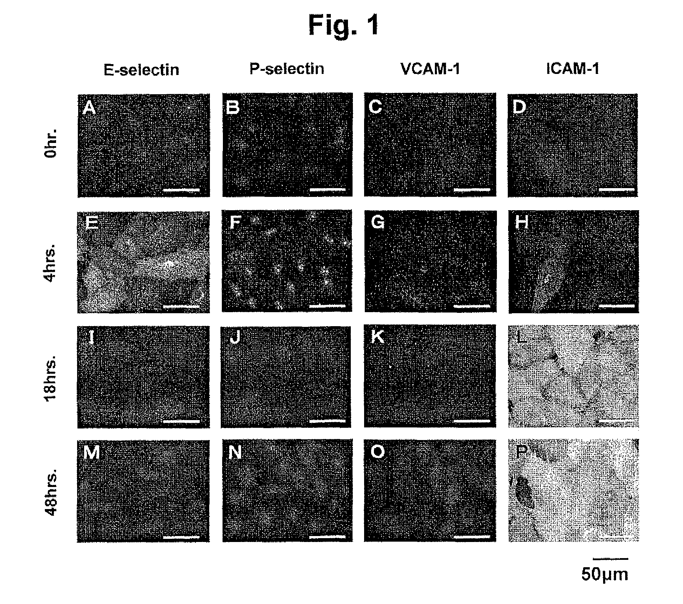

Fig. 1 shows representative microphotographs that indicate results of immunohistochemical expression of the adhesion molecules as E-selectin, P-selectin, VCAM-1 and ICAM-1 on the human LECs when stimulated with the supernatant of the culture medium of MDA-MB-231. After the human LECs were cultured on starvation medium containing 3% FBS overnight, and as shown in Fig.1 A, B, C and D, no or little immunoreactive staining of all adhesion molecules of E-selectin, P-selectin, VCAM-1 and ICAM-1 was observed on the cultured LECs when stimulated for 0 hr. Thus, no or little expression of adhesion molecules on the human LECs was caused. Similar to this observation, after the human LECs were cultured on DMEM/F12 culture medium with 3% FBS, no expression of those adhesion molecules on the human LECs was observed.

In contrast, as shown in Fig. 1E and F, when the human LECs were stimulated with the supernatant of the cell culture medium of MDA-MB-231 for 4 hrs., marked immunoreactive staining by E-selectin antiserum and P-selectin antiserum on most cultured human LECs was clearly observed respectively. Thus, the stimulation thereof caused a marked expression of E-selectin and P-selectin on the human LECs. As shown in Fig. 1G and H, when the human LECs were stimulated with the supernatant of cell culture medium of MDA-MB-231 for 4 hrs., only slight immunoreactive staining by VCAM-1 antiserum and ICAM-1 antiserum was observed respectively. Thus, the stimulation thereof caused only little expression of VCAM-1 and ICAM-1 on the human LECs.

However, by increasing the stimulation time to 18 hrs. and 48 hrs., the immunoreactions of anti-E-selectin and anti-P-selectin were markedly decreased as shown in Fig. 1 I, J, M and N. In contrast, as shown in Fig.1 L and P, all cultured cells in only case of ICAM-1 were strained strongly and the intensity of immunoreactivity for ICAM-1 was significantly increased, therefore the stimulation thereof caused sufficient expression of ICAM-1 on the human LECs.

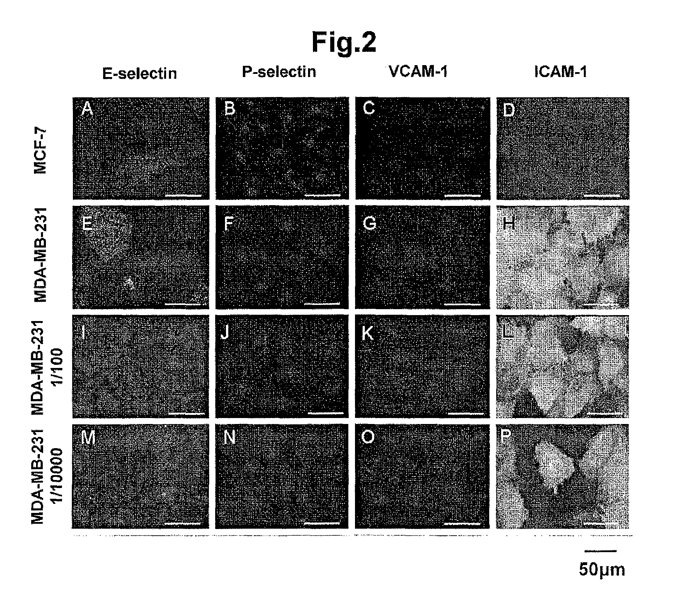

Fig. 2 shows representative microphotographs that indicate results of immunohistochemical expression of the adhesion molecules as E-selectin, P-selectin, VCAM-1 and ICAM-1 on the human LECs when stimulated with the supernatants of MDA-MB-231 or MCF-7 for 48 hrs.

As shown in Fig. 2 A, B, C and D, when the cultured human LECs were stimulated with the MCF-7 supernatant for 48 hrs., no or little immunoreactive staining in a case of E-selectin, P-selectin, VCAM-1 and ICAM-1 was observed on the human LECs. Thus, no or little expression of those adhesion molecules was caused on the human.

On the other hand, when the human LECs were stimulated with the MDA-MB-231 supernatant for 48 hrs., as shown in Fig.2 E, slight immunoreactive staining in a case of E-selectin was observed on the human LECs. Thus slight expression of E-selectin was caused on the human. And when the MDA-MB-231 supernatant was diluted to 1/100 times and 1/10,000 times respectively, as shown in Fig. 2I and M, little immunoreactive staining thereof was observed on the human LECs. And as shown in Fig.2F and G, little expression of P-selectin and VCAM-1 was caused on the human.

However, in contrast, when the human LECs were stimulated with the MDA-MB-231 supernatant for 48 hrs., as shown in Fig.2 H, immunoreactive staining by ICAM-1 antiserum was clearly observed on almost cultured human LECs. Thus, marked expression of ICAM-1 was significantly caused on the human LECs. Furthermore when the human LECs were stimulated with the supernatant diluted by 1/10,000 times, as shown in Fig. 2 P, immunoreactivity thereof was fairly reduced but still strong immunoreactive staining was observed. Thus, the overexpression of ICAM-1 to that produced by the diluted supernatant was caused on the human LECs.

### (5.2 Measurements of cytokines and growth factors in supernatant of MDA-MB-231 or MCF-7)

Table 1 shows summarized concentration data of cytokines measurements: TNF-α, TGF-β1, INF-γ, IL-1β, IL-6, IL-12, and growth factors measurements; bFGF, PDGF-BB, VEGF-A, and VEGF-C in the supernatant of culture medium of MDA-MB-231 or MCF-7.

**Table 1**

| | MCF-7 | MDA-MB-231 |
|---|---|---|
| TNF-α (pg/mL) | <5 | <5 |
| TGF-β (ng/mL) | <0.5 | <0.5 |
| IFN-γ (IU/mL) | 0.6 | 0.5 |
| IL-1β (pg/mL) | 18 | 18 |
| IL-6 (pg/mL) | 1.7 | 195 |
| IL-12 (pg/mL) | <7.8 | <7.8 |
| bFGF (pg/mL) | <10 | <10 |
| PDGF-BB (pg/mL) | <31.2 | <31.2 |
| VEGF-A (pg/mL) | 334 | 1150 |
| VEGF-C (pg/mL) | <109 | 554 |

AS shown in Table 1, the concentrations of IL-6, VEGF-A and VEGF-C in the MDA-MB-231 supernatant were significantly higher than those obtained with the MDA-MB-231 supernatant.

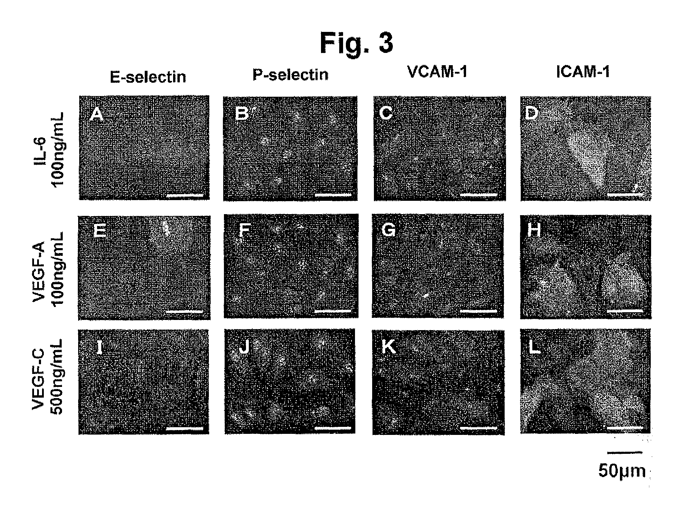

Thus, the effects of 48 hrs. treatment with 100ng/mL IL-6, 100ng/mL VEGF-A, or 500ng/mL VEGF-C on the expression of adhesion molecules on the human LECs., were examined. The data with representative microphotographs are summarized in Fig. 3. In contrast to the data obtained with the MDA-MB-231 supernatant, almost all cultured LECs were strongly stained by P-selectin antiserum (Fig. 3 B, F, and J). Slight staining with VCAM-1 and ICAM-1 was also found on the human LECs (Fig. 3 C, D, G, H, K, and L). No or little expression of E-selectin was observed on the LECs (Fig. 3 A, E, I).

### (5.3 Measurement of ATP in MDA-MB-231 supernatant)

The concentrations of ATP in the MDA-MB-231 supernatant or the culture medium in the absence or presence of ATP (10⁻⁸, 10⁻⁷, and 10⁻⁶M) are summarized in Table 2.

**Table 2**

| | Relative Luminescence Intensity |
|---|---|
| ATP 10⁻⁸ M | 1138.5 ± 47.5 |
| ATP 10⁻⁷ M | 9679.0 ± 1193.2 |
| ATP 10⁻⁶ M | 67462.0 ± 947.1 |
| MDA-MB-231 Supernatant | 1794.0 ± 115.0 |
| Culture Medium Only | 365.0 ± 37.0 |

Linear regression analysis of the culture medium containing with 10⁻⁸,10⁻⁷, and 10⁻⁶ M ATP in Table 2 suggests that the concentration of ATP in the MDA-MB-231 supernatant is about 2.1 x 10⁻⁸M.

### (5.4 Effects of enzymatic digestion with protease, heating, or dialysis of MDA-MB-231 supernatant on expression of adhesion molecules on human LECs)

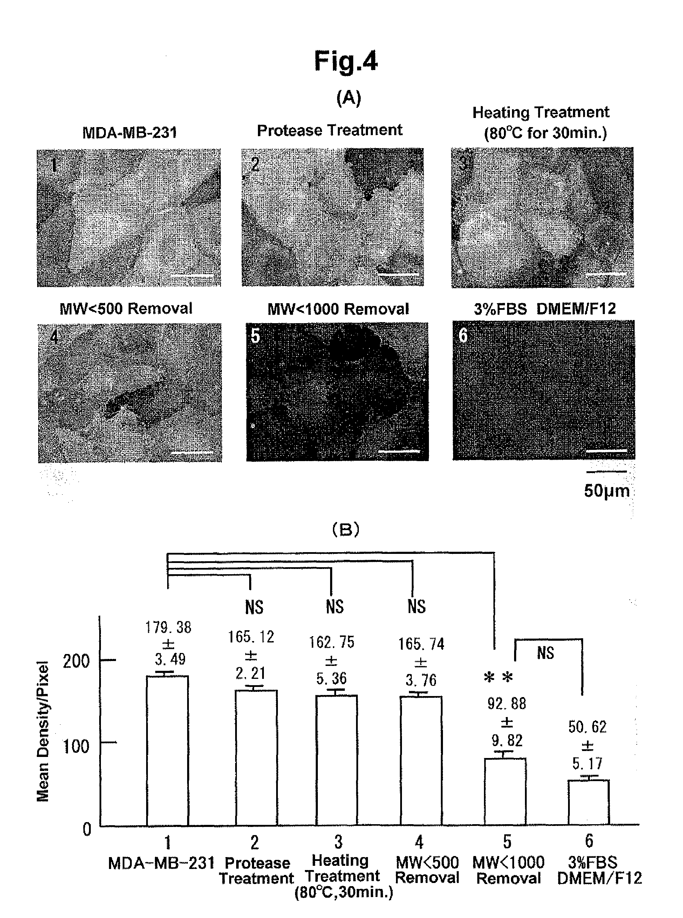

As shown in Fig. 4A-1 to A-4, after pretreatment of the MDA-MB-231 supernatant with the enzymatic digestion by protease or heating as well as pretreatment with the dialysis membrane for removing molecules having less than 500 of molecular weight from the supernatant, those pretreatment had no significant effect on the supernatant-mediated expression of ICAM-1 on the human LECs. And as shown in Fig. 4B-1 to B-4, there are no significant differences between cases using the MDA-MB-231 supernatant with and without the pretreatment. Incidentally number of test samples of above-mentioned measurement (i.e. n) is all 5.

In contrast, the pretreatment with the dialysis membrane for removing molecules having less than 1,000 of molecular weight from the MDA-MB-231 supernatant significantly reduced the expression of ICAM-1 on the human LECs. Thus, the immunohistochemical expression of ICAM-1 on the LECs is reduced by quite similar level to the expression of ICAM-1 produced by normal culture treatment for 48 hrs. with culture medium of DMEM/F12 with 3% FBS (negative control), as shown in Fig. 4A-6. There are significant differences (p<0.01) between cases using the MDA-MB-231 supernatant without and with pretreatment of dialysis membrane for removing molecules having less than 1,000 of molecular weight from the MDA-MB-231 supernatant, as shown respectively in Fig. 4B-1 and B-5, while there are no significant differences between pretreatment with the dialysis membrane and the negative control, as shown respectively in Fig. 4B-5 and B-6. Incidentally n is 5 respectively.

### (5.5 Effect of suramin on ATP-mediated expression of ICAM-1 on human LECs)

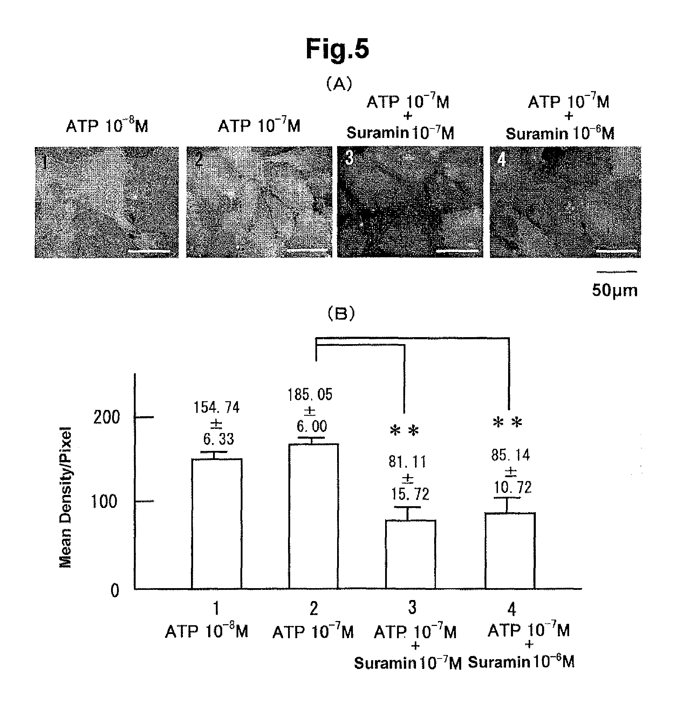

In agreement with the evidence that the molecular weight of ATP is 551.1, the effect of ATP on the expression of adhesion molecules on the human LECs was investigated. The results are shown in Fig. 5A-1 and A-2. Treatment with 10⁻⁸ or 10⁻⁷M ATP for 48 hrs. caused a marked expression of ICAM-1 on the human LECs, quite similar to the ICAM-1 expression on the LECs produced by treatment with the MDA-MB-231 supernatant for 48 hrs.

In contrast, simultaneous treatment with 10⁻⁷M or 10⁻⁶M suramin caused a significant reduction of the ATP-mediated expression of ICAM-1 on the LECs, as shown respectively in Fig. 5A-3 and A-4. And there are significant differences (p<0.01) between treatment with suramin and the treatment with 10⁻⁷M ATP, as shown respectively in Fig. 5B-3 or B-4 and B-2. Incidentally n is 5 respectively.

### (5.6 Effect of suramin, DPCPX or DMPX on MDA-MB-231 supernatant-mediated expression of ICAM-1 on human LECs)

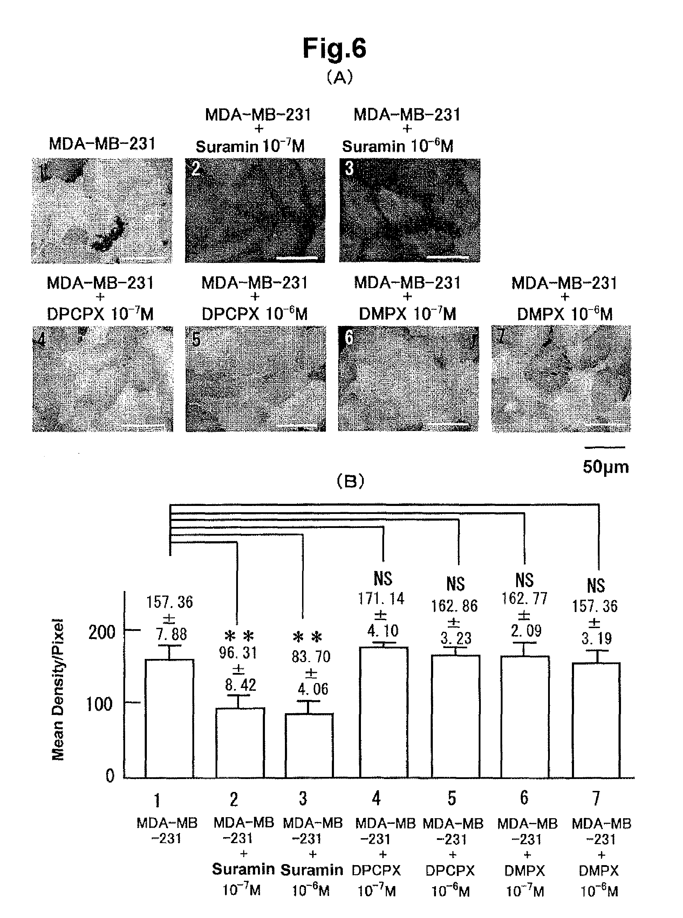

As shown in Fig. 6A-1, A-2 and A-3, the 48 hrs. simultaneous treatment of the MDA-MB-231 supernatant with 10⁻⁷ or 10⁻⁶M suramin caused a significant reduction of the MDA-MB-231 supernatant-mediated expression of ICAM-1 on the human LECs. Also, there were significant differences (p<0.01) between cases using the MDA-MB-231 supernatant with and without the simultaneous treatment by suramin, as shown respectively in Fig. 6B-1 and B-2 or B-3. Incidentally, n is 5 respectively.

In contrast, as shown in Fig. 6A-4 to A-7, 48 hrs. simultaneous treatment of the MDA-MB-231 supernatant with DPCPX (10⁻⁷ or 10⁻⁶M) or DMPX (10⁻⁷ or 10⁻⁶M) produced no significant effect on the MDA-MB-231 supernatant-mediated expression of ICAM-1 on the human LECs. There was no significant difference between cases using the MDA-MB-231 supernatant with and without the simultaneous treatment by DPCPX or DMPX, as respectively shown in Fig. 6B-4 to B-7. Incidentally, n is 5 respectively.

### (5.7 Attachment assay with 48 hrs. stimulation of ATP or MDA-MB-231 supernatant in presence or absence of suramin)

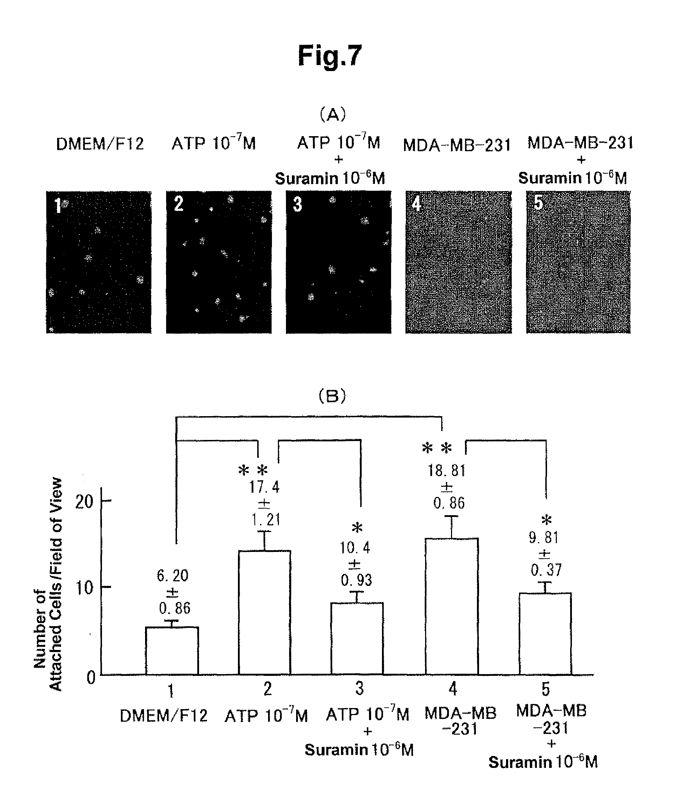

As shown in Fig. 7A-1, A-2 and A-3, 48 hrs. stimulation of 10⁻⁷M ATP caused a significant increase of the in vitro attachment of carcinoma cells to the human LECs compared to that of DMEM/F12 Ham culture medium as a negative control. In contrast, the increased attachment of carcinoma cells to the LECs was significantly reduced by simultaneous treatment with 10⁻⁶M suramin. There were significant differences (p<0.01) between a case using ATP for the stimulation treatment as shown in Fig. 7B-2 and a case without using ATP by normal culture treatment in DMEM/F12 Ham culture medium as shown in Fig. 7B-1 or a case with using ATP and suramin for simultaneous treatment as shown in Fig 7B-3. Incidentally n is 5 respectively.

Similar to the stimulation of ATP, as shown in Fig. 7A-4 and A-5, 48 hrs. treatment with MDA-MB-231 supernatant produced significant facilitation of the in vitro attachment of the carcinoma cells on the human LECs. In addition, 48 hrs. simultaneous treatment with 10⁻⁶M suramin caused a significant reduction of the MDA-MB-231 supernatant-mediated increase of the in vitro attachment of carcinoma cells to the human LECs. There were significant differences (p<0.01) between a case using MDA-MB-231 supernatant for the stimulation treatment as shown in Fig. 7B-4 and a case without using MDA-MB-231 supernatant by normal culture treatment in DMEM/F12 Ham culture medium of the negative condition as shown in Fig. 7B-1 or a case with using MDA-MB-231 supernatant and 10⁻⁶M suramin for simultaneous treatment as shown in Fig 7B-5. Incidentally, n is 5 respectively.

### (5.8 Attachment assay with 48 hrs. stimulation of ATP or MDA-MB-231 supernatant in present or absence of anti-ICAM-1 antibody)

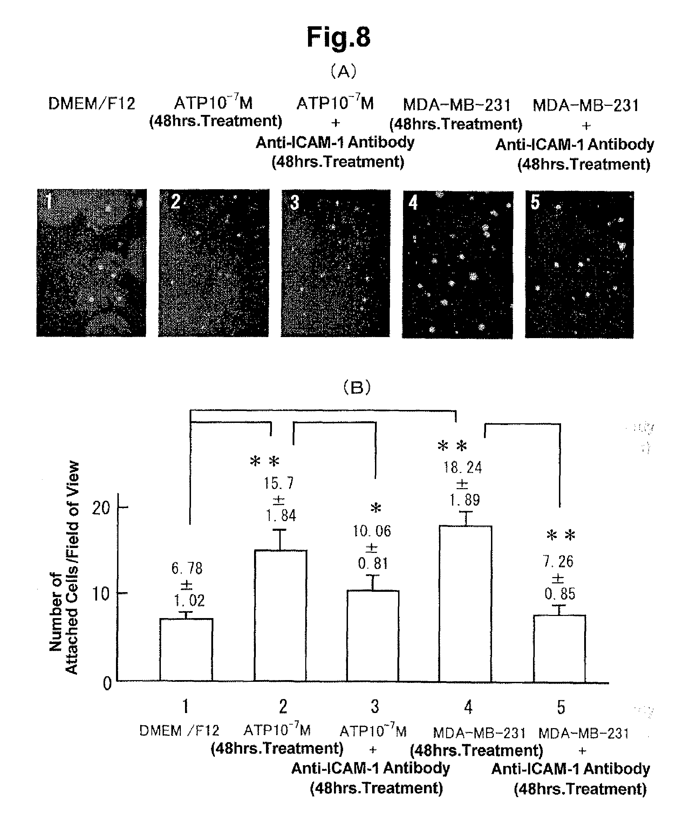

Next, it was examined whether the MDA-MB-231 supernatant or ATP mediating the facilitation of the in vitro attachment of the carcinoma cells to the human LECs can be blocked by treatment with ICAM-1 antibody. As shown in Fig. 8A-1 to A-3, the 48 hrs. stimulation of 10⁻⁷M ATP produced a significant increase of the attachment of the carcinoma cells to the human LECs in comparison to that of DMEM/F12 Ham culture medium as the negative control. The 10⁻⁷M ATP-mediated increase of the attachment assay was significantly reduced by treatment with anti-ICAM-1 antibody. There were significant differences (p<0.01) between a case using the ATP for the stimulation treatment as shown in Fig. 8 B-2 and a case without using ATP by normal culture treatment in DMEM/F12 Ham culture medium of the negative condition as shown in Fig. 7 B-1 or a case with using it and anti-ICAM-1 antibody for the treatment as shown in Fig. 8 B-3. Incidentally, n is 5 respectively.

Similar to the stimulation of ATP, as shown in Fig. 8 A-4 and A-5, 48 hrs. stimulation of the MDA-MB-231 supernatant also caused a significant increase of the in vitro attachment of the carcinoma cells to the human LECs in comparison to that of DMEM/F12 Ham culture medium as a negative control. The increased attachment of the carcinoma cells to the human LECs was significantly reduced by additional treatment with anti-ICAM-1 antibody. Additionally, 48 hrs. simultaneous treatment with 10⁻⁶M suramin caused a significant reduction of the MDA-MB-231 supernatant-mediated increase of the in vitro attachment of the carcinoma cells to the human LECs. There were significant differences (p<0.01) between a case using the MDA-MB-231 supernatant for the stimulation treatment as shown in Fig. 8 B-4 and a case without using MDA-MB-231 supernatant by normal culture treatment in DMEM/F12 Ham culture medium of the negative condition as shown in Fig. 8 B-1 or a case with using it and anti-ICAM-1 antibody for the treatment as shown in Fig. 8 B-5. Incidentally, n is 5 respectively.

### (5.9 Immunohistochemical expression of lymph vessel markers on cultured human LECs)

Fig. 9 shows representative microphotographs of lymph vessel markers such as VEGF R3 (Fig. 9C), LYVE-1 (Fig. 9D), Prox-1 (Fig. 9E) and podoplanin (Fig. 9F) on the cultured cells. The cultured cells were strongly stained by VEGF R3, Prox-1, podoplanin and PECAM-1 (Fig. 9B) antisera. In contrast, the antibody to LYVE-1 weakly stained only a few of the cultured cells (Fig. 9D). Fig. 9A shows a representative microphotograph of phase contrast images of the cultured cells. The results suggest that the cultured cells may be the human LECs in nearest afferent lymph vessels of the SLNs in patients with breast cancer.

### (5.10 Effect of chemokines on expression of adhesion molecules on human LECs)

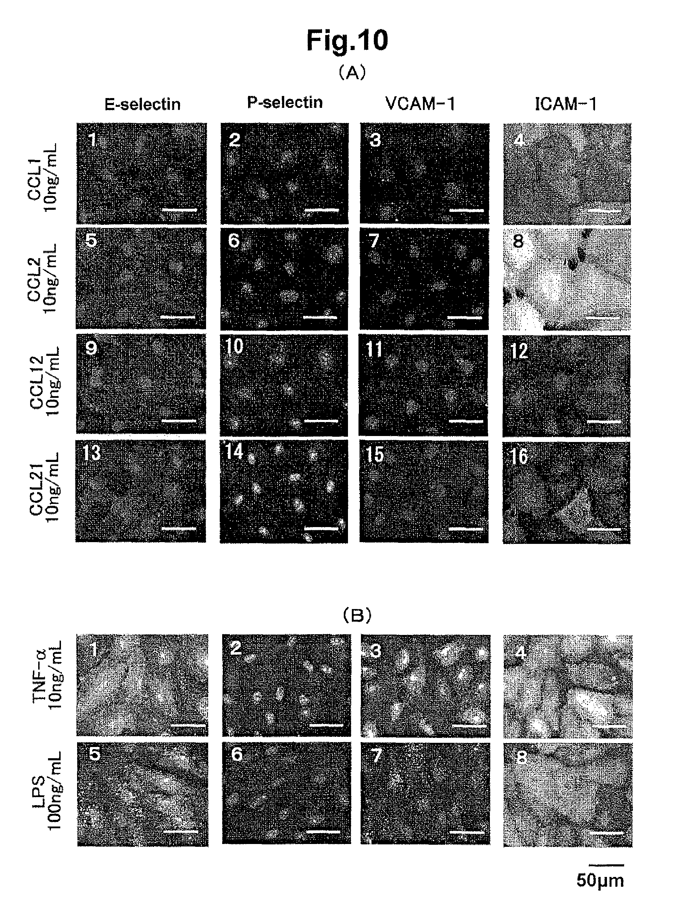

The effect of chemokines on the expression of the adhesion molecules on the human LECs is shown in Fig. 10. Fig. 10A shows representative microphotographs of the effects of 18 hrs.-stimulation on the cultured human LECs with various chemokines of 10ng/mL CCL1 (Fig. 10A 1-4), 10ng/mL CCL2 (Fig. 10A 5-8), 10ng/mL CCL12 (Fig. 10A 9-12) or 10ng/mL CCL21 (Fig. 10A 13-16) on the immunohistochemical expression of the adhesion molecules on the cultured human LECs. As shown in Fig. 10A 8, the 18 hrs.-stimulation with 10ng/mL CCL2 only caused a marked expression of ICAM-1 on the LECs. Thus, almost all cultured LECs were strongly stained by ICAM-1 antiserum (Fig. 10A 8). Little or no expression of E-selectin or VCAM-1 was observed on the cultured LECs (Fig. 10A 1, 5, 9, 13 and Fig. 10A 3, 7, 11, 15). In contrast, slight staining was observed with ICAM-1 antiserum on the LECs stimulated with 10ng/mL CCL1, 10ng/mL CCL12 or 10ng/mL CCL21 (Fig. 10A 4, 12, 16).

On the other hand, for positive controls of E-selectin, P-selectin and VCAM-1, the effects of TNF-α or LPS on the immunohistochemical expressions of the adhesion molecules on the cultured human LECs were examined. As shown in Fig. 10B, 18 hrs.-stimulation with 10ng/mL TNF-α produced marked expression of E-selectin (Fig. 10B 1), P-selectin (Fig. 10B 2), VCAM-1 (Fig. 10B 3) and ICAM-1 (Fig. 10B 4) on the LECs. In contrast, 18 hrs.-stimulation with 100ng/mL LPS caused a marked expression of E-selectin (Fig. 10B 5) and ICAM-1 (Fig. 10B 8), but no or little expression of P-selectin (Fig. 10B 6) and VCAM-1 (Fig. 10B 7) on the LECs.

Incidentally each microphotographs in Fig. 10 A and B were merged with the corresponding DAPI counterstaining image of the human LECs

### (5.12 Effects of stimulation time on CCL2-mediated expression of adhesion molecules or ICAM-1 mRNA on human LECs)

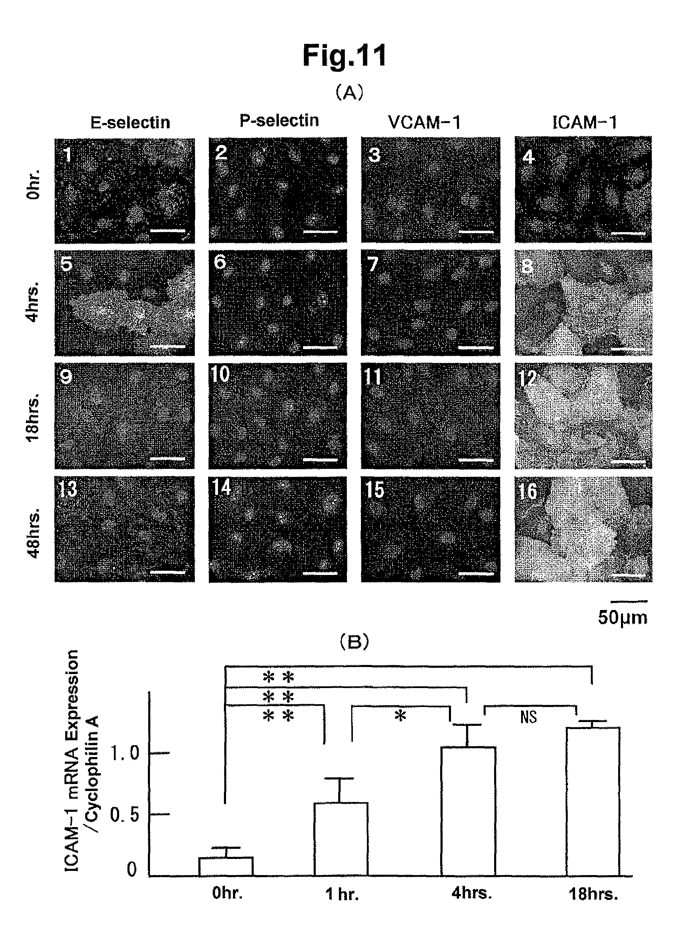

Fig. 11A shows the effects of stimulation time on the CCL2-mediated immunohistochemical expression of the adhesion molecules on the cultured human LECs. Fig. 11A 1-16 are representative microphotographs of the effects of the stimulation time (0 hr., 4 hrs., 18 hrs., and 48 hrs.) on the 10ng/mL CCL2-mediated immunohistochemical expression of the adhesive molecules of E-selectin (1, 5, 9, 13), P-selectin (2, 6, 10, 14), VCAM-1 (3, 7, 11, 15), and ICAM-1 (4, 8, 12, 16) on the cultured human LECs.

As shown on the microphotographs obtained at 0 hr., no or little expression of E-selectin, P-selectin, VCAM-1 or ICAM-1 was observed on the cultured LECs (Fig. 11A 1, 2, 3, 4). Thus, this overnight culture of starvation medium containing 3% FBS caused no or little expression of adhesion molecules on the LECs. Similar to this observation, 18 hrs.-culture of EBM-2 containing 3% FBS also produced no significant expression of adhesion molecules on the human LECs.

In contrast, 4hrs-stimulation of 10ng/mL CCL2 caused the marked expression of ICAM-1 on the cultured LECs (Fig. 11A 8). Thus, almost all cultured LECs were markedly stained by ICAM-1 antiserum. Slight staining with E-selectin antiserum was also observed on the LECs (Fig. 11A 5). On the other hand, no or little expression of VCAM-1 was found on the LECs (Fig. 11A 7). By increasing the stimulation time to 18 hrs. and 48 hrs., the immunoreaction of anti-E-selectin was markedly decreased (Fig. 11A 9, 13). However, the intensity of the immunoreactivity for ICAM-1 was significantly increased at the stimulation time of 18 hrs. only (Fig. 11A 12). Thus, after 18 hrs. of stimulation the immunoreactivity of ICAM-1 was found to be dense on all cultured LECs. Fig. 11B shows summarized data of the effects of stimulation time of 10ng/mL CCL2 on ICAM-1 mRNA levels in the cultured human LECs. The CCL2-mediated expression of ICAM-1 mRNA is significantly increased at 1 hr. after the stimulation. Also Fig. 11B shows the effects of stimulation time (0 hr., 1 hr., 4 hrs., and 18 hrs.) of 10ng/mL CCL2 on ICAM-1 mRNA levels in the human LECs evaluated by Reverse Transcription Polymerase Chain Reaction (RT-PCR). In Fig. 11B, the "**" denotes significantly different (p<0.01) and the "*" denotes significantly different (p<0.05), and NS denotes no significant differences, when each column was compared. The CCL2-mediated expression of ICAM-1 mRNA is significantly increased at 1 hr. after the stimulation. The increase of CCL2-mediated expression of ICAM-1 mRNA was kept around 4 hrs. after the stimulation, being maximal level of the expression. The CCL2-mediated expression of ICAM-1 mRNA increased slightly up to 18 hrs. after the stimulation.

### (5.13 Effects of the concentration of CCL2 on immunohistochemical expression of ICAM-1 on human LECs)

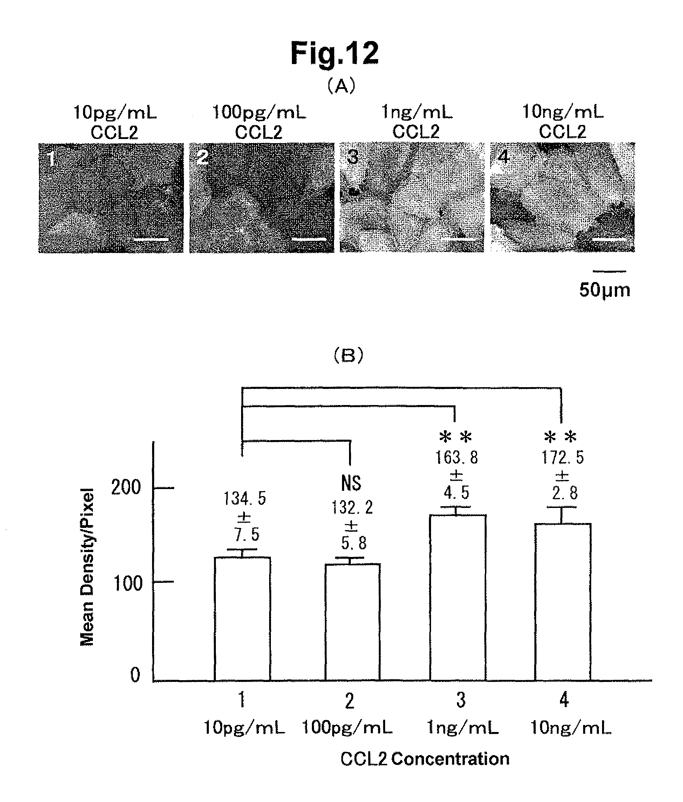

Fig. 12 shows the effects of the concentration of CCL2 between 10pg/mL and 10ng/mL on the immunohistochemical expression of ICAM-1 on the human LECs. Fig. 12A shows representative microphotographs of effects of 18 hrs.-stimulation on the cultured human LECs with various concentration of CCL2 of 10pg/mL (Fig. 12A 1), 100pg/mL (Fig. 12A 2), 1ng/mL (Fig. 12A 3) and 10ng/mL (Fig. 12A 4) for stimulation on the immunohistochemical expression of ICAM-1 on the human LECs. As shown Fig. 12A, 1 10pg/mL CCL2 caused a slight, but significant, expression of ICAM-1 on the cultured human LECs. The CCL2-mediated expression of ICAM-1 on the LECs was dose-dependently increased up to 1ng/mL. Thus, 1ng/mL or 10ng/mL of CCL2 produced a marked expression of ICAM-1 on almost all cultured LECs.

Fig. 12B shows the summarized contrast-density measurement data of predefined area in each microphotograph of the human LECs samples (n=5, respectively) graphically, which are expressed using relative unit (mean density/pixel). The data were obtained by image conversion from the microphotograph image to the gray scale image, determination of contrast-density thereof, and Scion Image analysis thereof. In Fig. 12B, the axis of ordinate shows the normalized number of the contrast-density measurement by mean density/pixel (n=5). The "**" in Fig. 12B, denotes a significant difference (p<0.01), and "NS" denotes no significant difference. As shown in Fig. 12B, there are no significant difference between cases using 10pg/mL (Fig. 12B 1) and 100pg/mL (Fig. 12B 2) of CCL2. However there are significant differences (p<0.01) respectively between cases using 10pg/mL (Fig. 12B 1) and 1ng/mL (Fig. 12B 3) or 10ng/mL (Fig. 12B 4) of CCL2.

### (5.14 Effects of CCL2 neutralization on CCL2-mediated expression of ICAM-1 on human LECs)

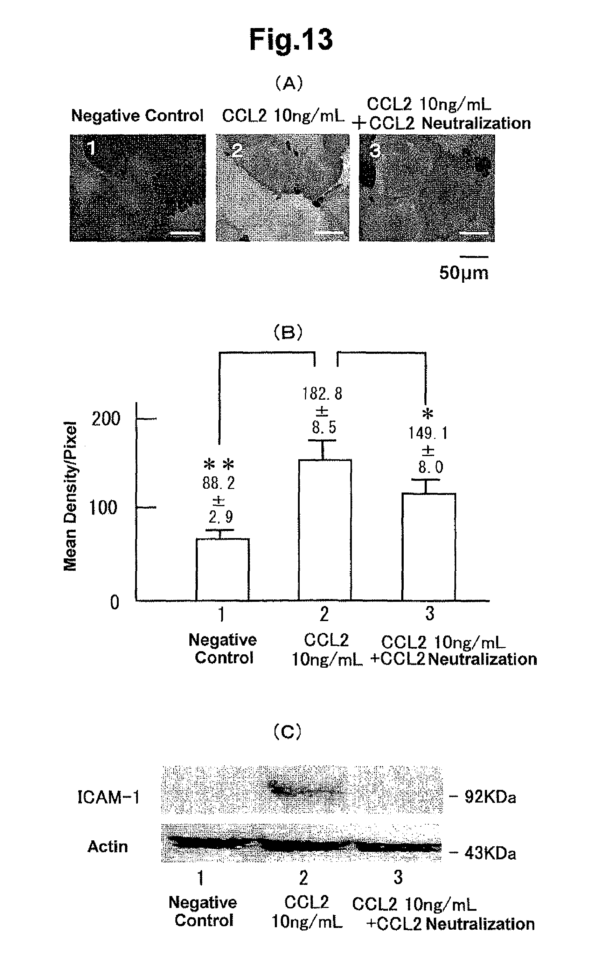

Fig. 13 demonstrates the effects of CCL2 neutralization on the CCL2-mediated expression of ICAM-1 on the cultured human LECs. Fig. 13A is representative microphotographs of the effects of 10ng/mL CCL2 in the presence (Fig. 13A 3) or absence (Fig. 13A 2) of 1.0µg/mL CCL2 specific antibody. Fig. 13A 1 is microphotograph of a negative control obtained with serum starvation cultured medium (EBM-2 containing 3% FBS). As shown in Fig. 13A 3, the neutralization of CCL2 with a specific CCL2 antibody caused a significant reduction of the CCL2-mediated immunohistochemical expression of ICAM-1 on the cultured human LECs.

Fig. 13B shows the summarized contrast-density measurement data of predefined area in each microphotograph of the human LECs samples (n=5, respectively) graphically, which are expressed using relative unit (mean density/pixel). The data were obtained by image conversion from the microphotograph image to the gray scale image, determination of contrast-density thereof, and Scion Image analysis thereof. The axis of ordinate denotes the same item as that in Fig. 12B. The "**" denotes a significant difference (p<0.01), and "*" denotes a significant difference (p<0.05), in Fig. 13B.

As shown in Fig. 13B, the contrast-density in case by treatment of 10ng/mL CCL2 increases significantly (p<0.01) in comparison with one of the negative control (Fig. 13B 1). And the contrast-density in case by neutralization after the treatment of 10ng/mL CCL2 decreases significantly (p<0.05) in comparison with one in case of only treatment of 10ng/mL CCL2.

Next, to analyze quantitatively the effect of CCL2 neutralization on the CCL2-mediated expression of ICAM-1 protein in the cultured human LECs, Western blot analysis was performed. Fig. 13C shows photographical partial result of the representative electrophoresis of Western blot analysis. As shown in Fig. 12C 2, 18 hrs.-stimulation of 10ng/mL CCL2 produced a significant expression of ICAM-1 protein in the cultured human LECs, whereas the CCL2-mediated expression of ICAM-1 protein was significantly inhibited by the treatment with CCL2 neutralization as shown in Fig. 13C 3. Incidentally Fig. 13C 1 shows a negative control.

### (5.15 Attachment assay after 18 hrs.-stimulation with addition of CCL2 neutralizer to 10ng/mL CCL2 in culture medium)

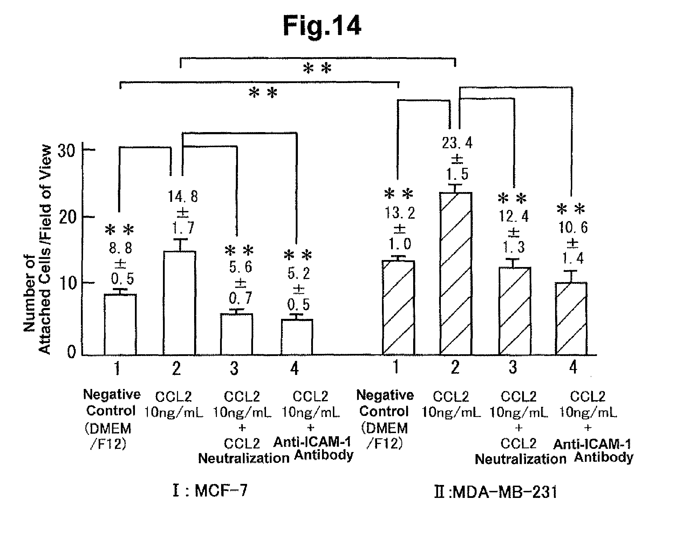

Attachment assay of counting carcinoma cells attached to the LECs was performed on culture medium with 10ng/mL CCL2 in the presence or absence of the neutralization of CCL2. Fig. 14 is the summarized graphical data of the attachment assay of the effects of 10ng/mL CCL2 in the absence (I-2 and II-2) or presence of CCL2 specific antibody (CCL2 neutralization: I-3 and II-3) or anti-ICAM-1 antibody (I-4 and II-4) using breast carcinoma cell lines MCF-7 (I) and MDA-MB-231 (II). The result of the attachment of carcinoma cells: MCF-7 is indicated with white column in Fig. 14 I, while that of carcinoma cells: MDA-MD-231 is indicated with diagonal hatching column in Fig.14 II. The axis of ordinate shows normalized number of the adherent carcinoma cells per field (x100). The "**" denotes a significant difference (p<0.01). As shown in Fig. 14 I-2 and II-2, the 18 hrs.-stimulation of 10ng/mL CCL2 caused a significant increase in the in vitro attachment of carcinoma cells, MCF-7 (I-2) and MDA-MB-231 (II-2), to the human LECs compared to negative control with DMEM/F12 (p<0.01). As shown in Fig. 14 I-3 and II-3, the increase in the attachment of carcinoma cells to the human LECs was significantly reduced by the neutralization of CCL2 after stimulation of 10ng/mL CCL2 in comparison to the case with 18 hrs.-stimulation of 10ng/mL CCL2 (p<0.01). Accordingly, 10ng/mL CCL2-mediated response is significantly different from each breast carcinoma cell.

### (5.16 Attachment assay with 18 hrs.-stimulation with 10ng/mL CCL2 in presence or absence of anti-ICAM-1 antibody)

Next, it was examined whether the 10ng/mL CCL2-mediated facilitation of the attachment of carcinoma cells, MFC-7 and MDA-MB-231, to the human LECs could be blocked by treatment with the ICAM-1 antibody. As shown in Fig. 14 I-4 and II-4, the increase in the attachment of carcinoma cells to the human LECs was significantly reduced by the treatment with the ICAM-1 antibody after 18 hrs. of in vitro stimulation with 10ng/mL CCL2 in comparison to the case with 18 hrs. of stimulation of 10ng/mL CCL2 (p<0.01).

### (5. 17 Immunohistochemical expressions of CD11a and CD11b on human breast carcinoma cell lines)

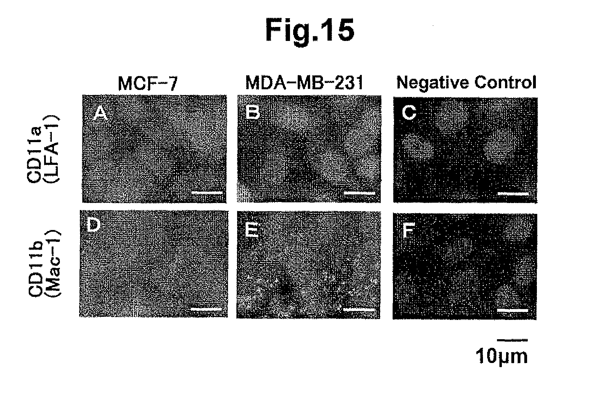

To evaluate counter receptors / ligands of ICAM-1, immunohistochemical expression of CD11a (LFA-1) and CD11b (Mac-1) on the human breast carcinoma cell lines, MCF-7 and MDA-MB-231 was examined. Fig. 15 shows representative microphotographs of the immunohistochemical expression of CD11a (Fig. 15 A, B) and CD11b (Fig. 15 D, E) on the human breast carcinoma cell lines, MCF-7 and MDA-MB-231. Fig. 15 C and F show representative microphotographs as negative controls without primary antibodies of CD11a and CD11b respectively. Immunohistochemical expression of both CD11a and CD11b were strongly observed on the MCF-7 and MDA-MB-231 cells.

### (5.18 Immunohistochemical expressions of E-selectin and ICAM-1 on SLN tissues with or without metastasis of carcinoma cells)

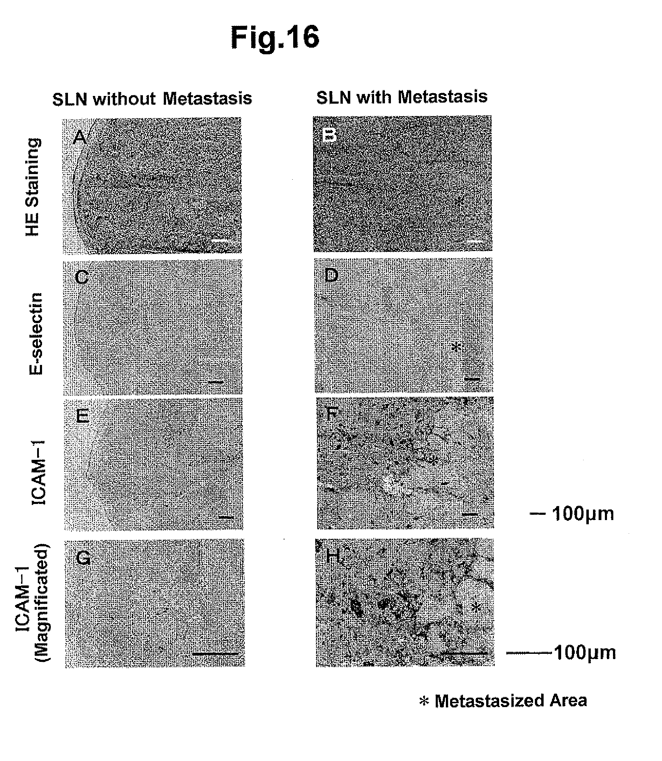

Fig. 16 demonstrates representative microphotographs of the immunohistochemical expressions of E-selectin (Fig.16 C and D) and ICAM-1 (Fig.16 E, F, G and H) on the fresh-frozen SLN tissues with the metastasis of the carcinoma cells isolated from the breast cancer patients and the fresh-frozen SLN tissues without metastasis of carcinoma cells isolated from the same patients. Fig.16 A and B are representative hematoxylin-eosin stained microphotographs of the SLN tissues without (Fig. 16 A) and with (Fig. 16 B) the metastasis of the carcinoma cells. As shown in Fig. 16 F and H, the immunohistochemical expressions of ICAM-1 were strongly observed on the SLN tissue with the metastasis of the carcinoma cells. In contrast, as shown in Fig. 16 E and G, the expression of ICAM-1 was weakly found on the SLN tissue without the metastasis of the carcinoma cells isolated from the same patient of breast cancer. On the other hand, as shown in Fig. 16 C and D, no or little expression of E-selectin was confirmed on the SLN tissues with and without the metastasis of the carcinoma cells. The "*" in Fig. 16 B, D, F and H denotes metastatic region of the carcinoma cells in the SLN.

It is emerged that the above-mentioned kit for detecting carcinoma cells metastasizing to sentinel lymph node and the drug delivery agent of the present invention are clinically useful as explained detail below.

### (6. Utility of kit for detecting carcinoma cells metastasizing to sentinel lymph node and drug delivery agent)

Regional lymph nodes are the most common and earliest site of metastasis of malignant tumors. Lymphatic nodes act as a physical barrier to prevent passage of carcinoma cells, and act as a biochemical barrier to inhibit growth of the tumor. Sentinel lymph node navigation surgeries achieve dramatically success in clinical practices. Therefore it is suggested that the regional lymph node has an efficacious filtering mechanism as the physical barrier against metastatic carcinoma cells. It was known that the primary tumor affects microenvironment though tumor tissue before serious metastases. However, it has been unclear what molecules in the regional lymph nodes develop a suitable environment for micro-metastasis within these lymph nodes before the metastases.

Meanwhile the inventors found that malignant tumors release key chemical substances that produce a microenvironment suitable for micrometastasis of carcinoma cells within regional lymph nodes, the inventors accomplished the kit for detecting carcinoma cells metastasizing to sentinel lymph node and the drug delivery agent of the present invention utilizing the findings. They are clinically useful as mentioned hereunder.

The major findings according to the present invention are summarized as follows.

### (6.1 Release of ATP from human breast carcinoma cell line: MDA-MB-231)

The supernatant of a malignant human breast carcinoma cell line with high metastatic ability, MDA-MB-231, caused the selective expression of ICAM-1 on the human LECs at 48 hrs. after the treatment. The intensity of the immunoreactivity of ICAM-1 was strong, despite x1/10,000 dilution of the supernatant; however, the supernatant of another human breast carcinoma cell line with low metastatic ability, MCF-7, produced no or little expression of ICAM-1 on the human LECs.

The concentrations of IL-6, VEGF-A, and VEGF-C in the MDA-MB-231 supernatant were significantly higher than those obtained from the MCF-7 supernatant; however, the cytokine and growth factors caused a slight expression of ICAM-1 on the human LECs, dissimilar to the MDA-MB-231 supernatant-mediated expression of ICAM-1 on the LECs.

Chemical treatment with dialyzed substances of <1,000 molecular weight caused a complete reduction of the MDA-MB-231 supernatant-mediated expression of ICAM-1 on the human LECs. In contrast, pretreatment with heating, enzymatic digestion of the MDA-MB-231 supernatant with protease, or chemical treatment with dialyzed substances of <500 molecular weight produced no significant effect on the supernatant-mediated expression of ICAM-1 on the human LECs. These findings suggest that the human breast carcinoma cell line MDA-MB-231 may release nonpeptide substances of >500 and < 1,000 molecular weight.

On the other hand, the concentration of ATP in the MDA-MB-231 supernatant was significantly higher than that obtained from the culture medium only. The effects of ATP on the expression molecules on the human LECs were investigated, and it was found that 10⁻⁸ and 10⁻⁷M ATP caused the same expression of ICAM-1 on the human LECs as that produced by the MDA-MB-231 supernatant.

Pretreatment with 10⁻⁷ and 10⁻⁶M suramin (a P2X and P2Y receptor antagonist) produced a significant reduction of ATP- and MDA-MB-231 supernatant-mediated expression of ICAM-1 on the LECs. The concentration of suramin is known to selectively block P2X and P2Y receptors.

In contrast, 10⁻⁷ and 10⁻⁶M DPCPX (a selective adenosine A₁ antagonist) or 10⁻⁷ and 10⁻⁶M DMPX (a selective adenosine A₂ antagonist) had no significant effect on the MDA-MB-231 supernatant-mediated expression of ICAM-1 on the human LECs.

Therefore, it is concluded that a malignant human breast carcinoma cell line, MDA-MB-231, may release or leak ATP, which can induce the selective expression of ICAM-1 on the human LECs through the activation of purinergic P2X and/or P2Y receptors on the LECs.

This conclusion is strongly compatible with other explained experimental findings that cytokines and growth factors such as IL-6, VEGF-A, and VEGF-C had no or little effect on the expression of ICAM-1 on the human LECs. This conclusion also agreed with evidence that the molecular weight of ATP is 551.1, between 500 and 1,000 of molecular weight. In addition, this conclusion may be strongly supported by inventor's previous physiological studies that a malignant melanoma cell line, B16-BL6, may release non-peptide substances of <1,000 molecular weight, resulting in significant cessation of lymphatic pump activity via the production and release of endogenous nitric oxide from lymphatic endothelial cells and the activation of mitochondrial ATP-sensitive K⁺ channels in lymphatic smooth muscle cells.

ATP also caused significant dilation with the cessation of lymphatic pump activity. ATP-induced dilation and inhibition of pump activity of isolated rat lymph vessels are endothelium-dependent and -independent responses. Thus, ATP-mediated inhibitory responses may be, in part, released to produce endogenous nitric oxide in lymphatic endothelium, or involve ATP-sensitive K⁺ channels in lymphatic smooth muscles. It is reasonable to hypothesize that a high concentration of ATP released or leaked out from malignant primary tumors, such as MDA-MB-231 and B16-BL6, diffuses the interstitial space, penetrates the lymph capillaries, modulates active lymph transport mechanisms, and then produces a premetastatic environment suitable for micrometastasis of carcinoma cells within regional lymph nodes. Thus, ATP causes dilation of lymph vessels and reduction of lymphatic pump activity, which may lead to decreased lymph flow, resulting in edema of the tumor tissues. Microenvironmental edema in the tumor tissues may affect the redistribution of tumor cells through regional initial lymph vessels, which may contribute, in part, to the occurrence of micrometastasis in sentinel lymph nodes.

### (6.2 ATP causes ICAM-1-mediated facilitation of attachment of carcinoma cells to human LECs)

As regards to another important aspect of the present findings, 48 hrs. treatment with MDA-MB-231 supernatant caused the significant facilitation of in vitro attachment of carcinoma cells to the human LECs. The stimulation of 10⁻⁷M ATP also produced a significant increase of the attachment of carcinoma cells to the LECs, the response to which is a quite similar to that produced by the MDA-MB-231 supernatant.

Both MDA-MB-231 supernatant- and ATP-induced responses were significantly reduced by simultaneous treatment with 10⁻⁶M suramin. The concentration of suramin is well known to selectively block purinergic P2X and P2Y receptors. Thus, the findings suggest that ATP facilitates the attachment of carcinoma cells to the human LECs nearest or within the SLNs through overexpression of the ICAM-1 adhesion molecule on the LECs via the activation of the purinergic P2X and/or P2Y receptors on the LECs.

Therefore, it is also concluded that a malignant human breast carcinoma cell line, MDA-MB-231, may release or leak large amounts of ATP, selectively inducing ICAM-1 adhesion molecule on the LECs nearest and/or within regional lymph nodes, and facilitating the attachment of carcinoma cells to the LECs. This conclusion may be strongly supported by the present findings that the ATP- or MDA-MB-231 supernatant-mediated facilitation of the attachment of carcinoma cells to the human LECs was significantly reduced by additional treatment with the anti-ICAM-1 antibody.

Thus, the ATP-mediated overexpression of ICAM-1 on the human LECs may contribute, in part, to build up the premetastatic environment and then produce micro-metastasis of the carcinoma cells within the regional lymph nodes.

In contrast, the supernatant of the human breast carcinoma cell line with low metastatic ability, MCF-7, caused no or little expression of ICAM-1 on the human LECs. Thus, there is marked heterogeneity between the carcinoma cells in the production and release of ATP that can modify micrometastasis of the carcinoma cells within the regional lymph nodes.

### (6.3 Pivotal roles of ICAM-1 in micrometastasis)

Recently, ICAM-1 expression by tumor cells has been reported to be a major contributing factor that facilitates metastatic progression. On the other hand, studies of leukocyte-endothelial cell adhesion tumor microvessels have revealed diminished adhesive interactions under both basal and cytokine-stimulated conditions. This observation is consistent with immunohistochemical and cytofluorimetric studies that predicted reduced endothelial ICAM-1 expression in tumor microvessels.

It has been suggested that the proposed downregulation of endothelial ICAM-1 facilitates tumor progression by allowing tumor cells to avoid immunosurveillance by circulating lymphocytes. There are, however, several other immunohistochemical studies of tumor vasculature that invoke the enhanced expression of endothelial ICAM-1, resembling an inflammatory phenotype, in non-small cell lung carcinoma and breast cancer. The expression of adhesion molecules on the human LECs remains unclear.

According to the present findings, MDA-MB-231 may have released or leaked ATP, which can produce the overexpression of ICAM-1 on the human LECs, and then facilitates the ICAM-1-mediated attachment of the carcinoma cells to the LECs located in the nearest SLN of the patients of the breast cancer.

The adhesion of leukocytes to the vascular endothelial cells is a critical step in the inflammatory response and involves the recruitment and infiltration of leukocytes to the site of tissue injury, infection, or lesion formation.

These processes are mediated by a wide variety of the adhesion molecules. ICAM-1 expressed on the endothelial cells is one of the major cell-surface glycoproteins that contribute to cell adhesion processes. Although ICAM-1 is constitutively expressed on the endothelial cells, it can be significantly induced on response to preinflammatory mediators such as tumor necrosis factor (TNF)-α and interleukin (IL)-1β.

The immunohistochemical findings using the kit for detecting carcinoma cells metastasizing to sentinel lymph node, suggested that ICAM-1 is not constitutively expressed on the lymphatic endothelial cells, which may be a characteristic property in comparison with the endothelial cells of the blood vessels. In addition, the concentrations of preinflammatory mediators, TNF-α and IL-1β, within the supernatant of the culture medium of MDA-MB-231, were measured. However no significant increase was confirmed to explain the overexpression of ICAM-1 on the human LECs.

### (6.4 Pivotal roles of ATP in oncology)

The anticancer activity of adenine nucleotides was already described. Intraperitoneal injection of ATP into tumor-bearing mice resulted in significant anticancer activity against several fast-growing aggressive carcinomas. ATP inhibits the growth of murine colonic adenocarcinoma and human pancreatic carcinoma in mice. Growth of prostate cancer cells in vitro is inhibited by up to 90% by ATP via P2 receptors, although it is not yet clear which subtype mediates this effect and whether it is a direct antiproliferative effect or a proapoptotic effect. Extracellular ATP suppressed the proliferation and induction of the differentiation of human HL-60 leukemia cells, partly mediated by adenosine and partly by ATP. P2X₇ receptor expression in the evaluative form of chronic lymphocyte leukemia has been identified; ATP decreased the proliferation of lymphocytes in this form of leukemia. The expression of P2X₇ receptor mRNA is higher in most types of leukemia, although there is loss of P2X₇ receptor function.

Recent studies have analyzed P2X receptor subtypes that contribute to ATP suppression of malignant melanomas in basal and squamous cell tumors and prostate and bladder cancers. In general, P2Y₁ and P2Y₂ receptors mediate proliferation or antiproliferation, and P2X₅ receptors mediate cell differentiation, which in antiproliferative and P2X₇ receptors in effect mediate apoptotic cell death.

In contrast, there exists no information, except for the present inventions, regarding the effects of ATP on the human LECs nearest and/or within sentinel lymph nodes with special reference to the expression of adhesion molecules and interaction with carcinoma cells such as the development of a premetastatic microenvironment and micrometastasis of carcinoma cells.

Therefore, it is suggested that ATP released and/or leaked out from malignant carcinoma cells with high metastatic ability may play crucial roles in the establishment of a premetastatic environment within the regional lymph nodes and the development of micrometastasis of carcinoma cells with high metastatic ability. The kit for detecting carcinoma cells metastasizing to sentinel lymph node and the drug delivery agent of the present invention, which may be clinically used, are originally utilized the above-mentioned suggestions.

### (6.5 CCL2 causes ICAM-1-mediated facilitation of attachment of carcinoma cells to human LECs)

Chemokines are soluble, small molecular-weight proteins that bind to their cognate G-protein coupled receptors to elicit cellular responses, usually directional migration or chemotaxis. Tumor cells secrete and respond to chemokines, which facilitate the tumor growth that is achieved by increased endothelial cell recruitment, subversion of immunological surveillance, and maneuvering of the tumoral leukocyte chemokine profile to skew immunoediting such that the chemokines released enable tumor growth and metastasis to distant sites.

The CXCL12-CXCR4 axis facilitates metastasis to distant organs, and the CCL21-CCR7 pair favors metastasis to lymph nodes. These two chemokine ligand-receptor systems are key mediators of tumor cell metastasis for several malignancies and as such provide key targets for chemotherapy.

Regional lymph nodes are the most common and earliest site of metastasis of malignant tumors. The dramatic clinical success of sentinel node navigation surgery suggests that the regional lymph node has an effective filtering function as a mechanical barrier against migrating cancer cells. On the other hand, it is well known that primary tumors influence the microenvironment of tumor tissue before metastasis. However, it is unclear which molecules in the prometastatic regional lymph nodes can make a suitable environment for micrometastasis within the nodes. Therefore, the inventors of the present invention have hypothesized that malignant tumors and/or metastatic carcinoma cells release key chemical substances that produce a microenvironment suitable for micro-metastasis of carcinoma cells within regional lymph nodes.

The chemokine CCL2, but neither CCL1, CCL12, nor CCL21 caused a selective and significant immunohistochemical expression of ICAM-1 in the cultured human LECs isolated from the nearest afferent lymph vessels of sentinel lymph nodes in patients with breast cancer. By increasing the stimulation time of CCL2 from 4 hrs. to 18 hrs. and 48 hrs., the intensity of the immunoreactivity for ICAM-1 was significantly increased dependent on the stimulation time up to 18 hrs. The ICAM-1 mRNA levels were also elevated significantly up to 18 hrs. The CCL2-mediated expression of ICAM-1 protein was also confirmed at 18 hrs.-stimulation by Western blot analysis. The CCL2-mediated immunohistochemical expression of ICAM-1 on the LECs was dose-dependently increased from 10pg/mL up to 1ng/mL. The CCL2-mediated expression of ICAM-1 on the human LECs was significantly reduced by the neutralization of CCL2 with a specific CCL2 antibody. 18 hrs. of treatment with CCL2 caused a significant facilitation of the in vitro attachment of carcinoma cells, MDA-MB-231 and MCF-7, to the human LECs. The CCL2-mediated response in the attachment assay was significantly reduced by the neutralization of CCL2, or by additional treatment with an anti-ICAM-1 antibody. Therefore, the inventors have concluded that CCL2, but neither CCL1, CCL12, nor CCL21, induces the selective expression of ICAM-1 mRNA and protein on the cultured human LECs and then facilitates in vitro attachment of carcinoma cells, MDA-MB-231 and MCF-7, to the cultured LECs through the overexpression of ICAM-1 in an in vitro micrometastatic experimental model. Thus, the CCL2-mediated overexpression of ICAM-1 on the human LECs may contribute, in part, to creating a suitable microenvironment and then developing micrometastasis of carcinoma cells within regional lymph node.

This conclusion may be compatible with a recent paper that showed that an intratumoral injection of CCL2 in mouse pancreatic cancer produced expression of ICAM-1 in the tumor tissues and then induces effective interaction between monocytes and endothelial cells in the peritumoral area. It is also known that CCL2 binds to specific receptors, mainly found on monocytes, and regulates monocyte behavior in inflammatory and cancer tissues. However, the assertion that monocyte/macrophage infiltration is an important aspect of host response in tumor growth remains controversial. Activated macrophages are known to be cytotoxic for cancer cells, but less so for normal cells. On the other hand, tumor-associated macrophages have been shown to promote the growth of tumor cells in vitro and to be positively correlated with tumor invasion and progression. Although the role of monocytes/macrophages in tumor tissues is controversial, monocyte migration via micro- and lymph-circulation to tumor sites would be necessary in host immune responses at least before advanced stages. In addition, the administration of specific chemokines for the recruitment of monocytes may trigger anti-tumor host responses.

It may be noteworthy to mention again that CCL2 produced an overexpression of ICAM-1 on the human lymphatic endothelial cells (LECs) in the nearest afferent lymph vessels, and/or within the sentinel lymph nodes (SLNs) and this facilitated interactions between the LECs and the carcinoma cells.

The concentrations of CCL2 in the supernatants of the culture medium of the MDA-MB-231 and MCF-7 cells were determined less than 62.5pg/mL by ELISA assay. It is known that lipopolysaccharide induces the expression of ICAM-1 and CCL2 on the cultured human LECs isolated from dermal micro lymph vessels. However, the source of CCL2 which causes an overexpression of ICAM-1 within the sentinel lymph node remains unclear.

### (6.6 Pivotal roles of ICAM-1 in micro metastasis)

ICAM-1 expression by tumor cells has been reported to be a major contributing factor that facilitates metastatic progression. On the other hand, the study of tumor microvessels with leukocytes-endothelial cell adhesion has revealed that adhesive interactions diminished under both basal and cytokine-stimulated conditions. This observation is consistent with immunohistochemical and cytofluorimetric studies that showed that reduced endothelial ICAM-1 expression is predicted in tumor micro vessels. Thus, it has been suggested that the proposed downregulation of endothelial ICAM-1 facilitates tumor progression by allowing tumor cells to avoid immunosurveillance by circulating lymphocytes. However, there are several other studies of tumor vasculature that invoke the enhanced expression of endothelial ICAM-1, resembling an inflammatory phenotype, in breast cancer. The expression of adhesion molecules on human LECs has remained unclear.

The inventors of the present invention found that CCL2 produced the overexpression of ICAM-1 on the human LECs, and then the facilitated ICAM-1 mediated attachment of the carcinoma cells to the LECs located in the nearest afferent lymph vessels of the sentinel lymph nodes in the patients with breast cancer, therefore the inventors accomplished the present invention. The counter receptors / ligands of ICAM-1 such as CD11A and CD11B were clearly observed on MDA-MB-231 and MCF-7: human breast carcinoma cells which were used in the in vitro attachment assay. In addition, the immunohistochemical expression of ICAM-1, but not E-selectin was strongly observed on the fresh-frozen SLN tissues with metastasis of carcinoma cells isolated from breast cancer patients. Therefore, this invention may be the first to suggest that CCL2 may play crucial roles in the development of the microenvironment within the regional lymph node for producing the micrometastasis of the carcinoma cells.

### Industrial Applicability

The kit for detecting carcinoma cells metastasizing to the sentinel lymph node of the present invention is useful for specifying the lymph nodes, which should be removed, before or during the surgery of removing the primary tumor.

And the drug delivery agent of the present invention can selectively deriver the drugs to the metastasized lymph nodes, therefore it is used as the marker or the quantitative agent for the medical treatment or the diagnosis thereof. Furthermore it can be derived to the lymph nodes attached the micro-metastasized carcinoma cell(s), therefore it is used for arresting or preventing the progress of malignant cancer.

## Claims

1. A kit for detecting carcinoma cells metastasizing to sentinel lymph node comprising an endothelial cell line derived from a human lymph vessel which is applied to a medium.

2. The kit for detecting carcinoma cells metastasizing to sentinel lymph node according to claim 1, wherein the endothelial cell line derived from the human sentinel lymph vessel is consisted of endothelial cells collected through abrasion by intraluminal circulation of protease solution in an extirpated human lymph vessel.

3. The kit for detecting carcinoma cells metastasizing to sentinel lymph node according to claim 1 comprises;
an immunoassay detecting agent, which detects an adhesive molecule mediated attachment of the carcinoma cells metastasized from a primary tumor with the endothelial cell line derived from the human lymph vessel by performing an antigen-antibody reaction.

4. The kit for detecting carcinoma cells metastasizing to sentinel lymph node according to claim 3, wherein the adhesive molecule is expressed by being activated.

5. The kit for detecting carcinoma cells metastasizing to sentinel lymph node according to claim 3, wherein the adhesive molecule is ICAM-1 or E-selectin.

6. The kit for detecting carcinoma cells metastasizing to sentinel lymph node according to claim 3, wherein the adhesive molecule is bonded with the carcinoma cells through a ligand.

7. The kit for detecting carcinoma cells metastasizing to sentinel lymph node according to claim 6, wherein the ligand is CD11a, CD11b and/or CD11c.

8. A drug delivery agent comprising;
an antibody and/or a ligand therein which are exposed on a surface of colloid particles in the agent or suspended in the agent,
wherein the antibody and/or the ligand are interacted with an adhesive molecule mediated attachment of carcinoma cells metastasized from a primary tumor with lymphatic endothelial cells in a sentinel lymph node.

9. The drug delivery agent according to claim 8, wherein the antibody is anti-ICAM-1 antibody and/or anti-E-selectin antibody.

10. The drug delivery agent according to claim 8, wherein the ligand is a ligand of ICAM-1.

11. The drug delivery agent according to claim 10, wherein the ligand is CD11a, CD11b and/or CD11c.

12. The drug delivery agent according to claim 8, wherein the colloid particles comprise and/or express a fluorescence agent, a contrast agent, a therapeutic agent and/or a potentiator for adhesion of the metastasized carcinoma cell.

13. The drug delivery agent according to claim 8, wherein the colloid particle is selected from the group consisting of micelle particles of a biodegradable resin, micelle particles of a synthetic resin or liposome.


[SEQUENCE LISTING]

<110>Shinshu University

<120>Kit for Detecting Metastasized Tumor in Sentinel Lymph Nodes

<130>L07-22PCT

<140>JP 2008-050735
<141>2008-02-29

<160>2

<210>1
<211>21
<212>DNA
<213>Artificial Sequence

<220>
<223>[forward primer]

<400>1
ttcgtgctct gagcactgga g                                21

<210>2
<211>24
<212>DNA
<213>Artificial Sequence

<220>
<223>[reverse primer]

<400>2
ggacccgtat gctttaggat gaag                                24

SEQUENCE LISTING

<110>Shinshu University

<120>Kit for Detecting Metastasized Tumor in Sentinel Lymph Nodes

<130>L07-22PCT

<140>JP 2008-050735
<141>2008-02-29

<160>2

<210>1
<211>21
<212>DNA
<213>Artificial Sequence

<220>
<223>[forward primer]

<400>1
ttcgtgctct gagcactgga g                                                21

<210>2
<211>24
<212>DNA
<213>Artificial Sequence

<220>
<223>[reverse primer]

<400>2
ggacccgtat gctttaggat gaag                                             24